# EUROPEAN PATENT APPLICATION

(11) **EP 2 592 077 A1**
(43) Date of publication of application: **15.05.2013**
(21) Application number: 11382343.9
(22) Date of filing: 11.11.2011
(51) Int. Cl.: C07D 403/12, C07D 409/12, C07D 409/14, C07D 413/14, C07D 417/14, A61K 31/4709, A61P 11/00

(54) **New cyclohexylamine derivatives having beta2 adrenergic agonist and M3 muscarinic antagonist activities**

(71) Applicant: Almirall, S.A., 08022 Barcelona (ES)
(72) Inventor: Aiguade Bosch, Jose, 08980 Sant Feliu de Llobregat (ES); Gual Roig, Silvia, 08980 Sant Feliu de Llobregat (ES); Prat Quiñones, María, 08980 Sant Feliu de Llobregat (ES); Puig Duran, Carlos, 08980 Sant Feliu de Llobregat (ES)
(74) Representative: Elzaburu Marquez, Alberto

(57) **Abstract**

The present invention relates to novel compounds having β2 adrenergic agonist and M3 muscarinic antagonist dual activity, to pharmaceutical compositions containing them, to the process for their preparation and to their use in respiratory therapies.

## Description

### FIELD OF THE INVENTION.

The present invention relates to novel compounds having β2 adrenergic agonist and M3 muscarinic antagonist dual activity. This invention also relates to pharmaceutical compositions containing them, process for their preparation and their use in respiratory therapies.

### BACKGROUND OF THE INVENTION.

Bronchodilator agents play an outstanding role in the treatment of respiratory disorders such as COPD and asthma. Beta-adrenergic agonists and cholinergic muscarinic antagonists are well established bronchodilator agents in widespread clinical use. Beta-adrenergic agonists currently used by the inhaled route include short-acting agents such as salbutamol (qid) or terbutaline (tid) and long-acting agents such as salmeterol and formoterol (bid). These agents produce bronchodilation through stimulation of adrenergic receptors on airway smooth muscle, reversing the bronchoconstrictor responses to a variety of mediators, such as acetylcholine. Inhaled muscarinic antagonists currently used include the short-acting ipratropium bromide or oxitropium bromide (qid) and the long-acting tiotropium (qd). These agents produce bronchodilation by reducing vagal cholinergic tone of airway smooth muscle. In addition to improve lung function, these agents also improve quality of life and reduce exacerbations. There are in the clinical literature a number of studies strongly demonstrating that the administration of a combination of a beta-2 agonist and a M3 antagonist is more efficacious for the treatment of COPD than either of the components alone (for example, van Noord, J.A., et al., Eur.Respir.J., 2005; 26: 214-222). Pharmaceutical compositions containing a combination of both types of bronchodilator agents are also known in the art for use in respiratory therapy. As an example, WO2009013244 discloses a medical composition containing salmeterol as beta-adrenergic agonist agent and tiotropium as antimuscarinic agent.

The class of beta2 adrenergic is well known and widely used by the persons skilled in the art, such as physicians, pharmacists or pharmacologists, for the treatment of respiratory disease, in particular asthma and chronic obstructive pulmonary disease (COPD) (Paul A. Glossop et al., Annual Reports in Medicinal Chemistry, 2007, 41, 237-248). Most of the beta2 adrenergic agonists are derivatives of natural catecholamines (e.g. epinephrine and norepinephrine) with which they share some common structural features, which are responsible for the similar interaction of these compounds with the beta 2 receptors ("Goodman & Gilman's The Pharmacological Basis of Therapeutics", 10th edition, chapter 10, pages 215-233, Textbook of respiratory medicine, third edition, Chpater 11, p. 267-272). In fact, most of the beta2 adrenergic agonist compounds have a general structure type that is present in the catechol (epinephrine and isoproterenol), namely an aminoethanol core flanked by an aryl group (J.R.Jacobsen, Future Mwedicinal Chemistry, 2011, 3 (13), 1607-1622). Examples of the aryl group that afford beta2 potency are but not limited to catechol, saligenin, formamide and 8-carbostyril groups (Paul A. Glossop et al., Annual Reports in Medicinal Chemistry, 2007, 41, 237-248).

Dual-phamacology muscarinic antagonists-beta2 agonist (MABA) molecules present an exciting new approach to the treatment of respiratory disease by combining muscarinic antagonism and beta2 agonism in a single entity. In the literature there have been disclosed various compounds having both muscarinic receptor antagonist and beta2-agonist activity (A.D. Hughes et al., Future Medicinal Chemistry, 2011, 3(13), 1585-1605). All of these molecules a great variety of covalent linker fragments between the M3 antagonist and the beta2 agonist moieties, indicating that the structure of the linker radical is not critical to preserve both activities, although such linker fragments have showed to be an important tool for mediating physical properties and potency at each target.

A single molecule possessing dual activity at muscarinic M3 and adrenergic β2 receptors (MABA) would therefore be desirable both in terms of efficacy and side-effects in the treatment of COPD. It would show also a relevant advantage in terms of formulation compared with the two-component combination. It also would be easier to co-formulate with other therapeutic agents such as inhaled anti-inflammatories to create triple therapy combinations. Thus there is a need for new compounds having both beta2 receptor agonist and muscarinic receptor antagonist activity and being suitable for the treatment of respiratory diseases, such as asthma and COPD.

### SUMMARY OF THE INVENTION.

The invention provides novel compounds that both β2 adrenergic receptor agonist and muscarinic receptor antagonist activities. Accordingly, there is provided a compound of formula (A), or pharmaceutically acceptable salts or N-oxides or solvates or deuterated derivatives thereof: 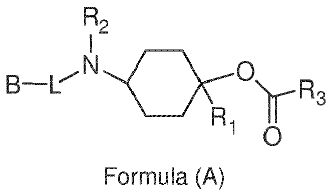 wherein • R₁ and R₂ independently are selected from the group consisting of a hydrogen atom, a linear or branched C₁₋₄ alkyl group, a linear or branched C₁₋₄ hydroxyalkyl group and a linear or branched C₁₋₄ alkoxy group,
- R₃ represents a group of formula: 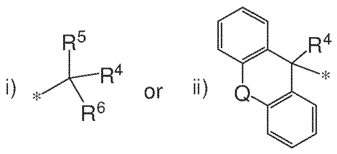
wherein:
○ R⁴ represents a hydrogen atom, a hydroxy group, a C₁₋₄ hydroxyalkyl group, a linear or branched C₁₋₄ alkyl group or a linear or branched C₁₋₄ alkoxy group,
○ R⁵ represents a saturated or unsaturated C₃₋₈ cycloalkyl group, a C₅₋₆ aryl group, a 5- to 6- membered heteroaryl group containing at least one heteroatom selected from N, S, and O; a (C₁₋₄ alkyl)-(C₅₋₆ aryl) group, a (C₁₋₄ alkyl)-(C₃₋₈ cycloalkyl) group or a (C₁₋₄ alkyl)-(5 to 6 membered heteroaryl group containing at least one heteroatom selected from N, S, and O) group, which groups independently are optionally substituted with one or more substituents R^{a},
○ R⁶ represents a C₅₋₆ aryl group, a 5- to 6- membered heteroaryl group containing at least one heteroatom selected from N, S, and O, a saturated or unsaturated C₃₋₈ cycloalkyl group, a linear or branched C₁₋₈ alkyl group, a C₂₋₈ alkenyl group, a C₂₋₈ alkynyl group, a linear or branched C₁₋₈ alkoxy group, a (C₁₋₄ alkyl)-(C₅₋₆ aryl) group, a (C₁₋₄ alkyl)-(C₃₋₈ cycloalkyl) group or a (C₁₋₄ alkyl)-(5- to 6- membered heteroaryl group containing at least one heteroatom from N, S, and O) group which groups independently are optionally substituted with one or more substituents R^{b},
○ R^{a} and R^{b} independently represent a halogen atom, a hydroxy group, a C₁₋₄ alkyl group, a C₁₋₄ alkoxy group, -SH, a C₁₋₄ alkylthio group, a nitro group, a cyano group, -CO₂R', -NR'R"', -C(O)NR'R", -N(R'")C(O)-R', -N(R'")-C(O)NR'R", wherein R', R" and R"' each independently represents a hydrogen atom or a C₁₋₄ alkyl group, or R' and " together with the nitrogen atom to which they are attached from a 3 to 6 membered heterocyclic ring,
o Q represents a direct bond, a -CH₂-, -CH₂-CH₂-, -O-, -O-CH₂-, -S-, -S-CH₂-, -NH-, -NH-CH₂- or-CH=CH-,
o * represents the point of attachment of R₃ to the remainder of the molecule of formula (A),
o L is a suitable covalent linker, and
o B is a moiety having a beta2-adrenergic binding activity,

L is a linker defined as a covalent bond between the beta2-agonist moiety B and the nitrogen atom of formula (A).

In one embodiment of the present invention, the linker L has the following formula (L): 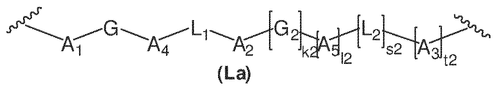 or 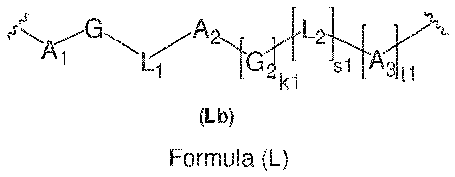 wherein k1, k2, s1, s2, 12, t1 and t2 independently have a value of 0 or 1;
- A₁, A₂, A₃, A₄ and A₅ each independently are selected from the group consisting of a C₂₋₁₀ alkylene group, a C₂₋₁₀ alkenylene group and a C₂₋₁₀ alkynylene group, wherein said groups are optionally substituted with one or more substituents selected from a halogen atom, a hydroxy group, a linear or branched C₁₋₄ alkyl group, a linear or branched C₁₋₄ alkoxy group, a C₅₋₆ aryl group and a C₃₋₇ cycloalkyl group,
- L₁ and L₂ independently are selected from a direct bond, -O-, -NR^{c}-, -S-, -S(O)-, SO₂-, -NR^{c}(CO)-, -(CO)NR^{c}-, -NR^{c}(CO)(CH₂)_{q}O-, -O(CH₂)q(CO)NR^{c}-, O(CH₂)qNR^{c}-, -NR^{c}(CH₂)_{q}O-, -NR^{c}(CO)NR^{d}-, -C(O)-, -C(O)O-, -OC(O)-, -S(O)₂NR^{c}-, -NR^{c}S(O)₂-, -NR^{c}S(O)₂NR^{d}-, -C(O)NR^{c}S(O)₂- and -S(O)₂NR^{c}C(O)-, wherein R^{c} and R^{d} are independently selected from a hydrogen atom and a linear or branched C₁₋₄ alkyl group and q has a value of 0, 1 or 2,
- G and G₂ independently are selected from the group consisting of a direct bond, a C₃₋₁₀ mono- or bicyclic cycloalkyl group, a C₅-C₁₄ mono- or bicyclic aryl group, a 3 to 14-membered saturated or unsaturated mono- or bicyclic heterocyclyl group having one or more heteroatoms selected from N, S and O, a 5- to 14-membered mono- or bicyclic heteroaryl group having one or more heteroatoms selected from N, S and O and a bicyclic ring system comprising two monocydic ring systems which are linked between each other by a covalent bond wherein said monocyclic ring systems are independently selected from a C₃₋₈ cycloalkyl group, a C₅₋₆ aryl group, a 3 to 8-membered saturated or unsaturated heterocyclyl group having one or more heteroatoms selected from N, S and O and a 5- to 6-membered heteroaryl group having one or more heteroatoms from N, S and O, wherein the cyclic groups independently are optionally substituted with one or more substituents selected from a halogen atom, a C₁₋₄ alkyl group, a C₁₋₄ alkoxy group, a carboxy group, a cyano group, a nitro group, a hydroxy group, an oxo group, a trifluoromethyl group and a trifluoromethoxy group.

In a preferred embodiment, all of k1, k2, s1, s2, 12, t1 and t2 have a value of 0

In a still preferred embodiment the linker L has the following formula (Lb1): 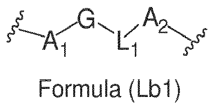 wherein A₁, A₂, L₁ and G are as defined above.

In a preferred embodiment, compounds of the present invention have the following formula (I):

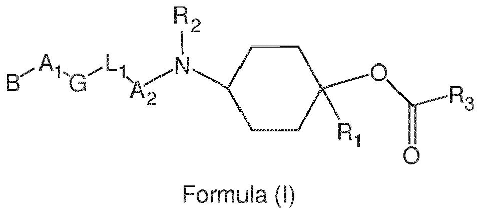

Wherein
- **A₁,** A₂, B and L₁ are as defiend above,
- R₁ and R₂ independently are selected from the group consisting of a hydrogen atom and a linear or branched C₁₋₄ alkyl group,
- R₃ represents a group of formula: 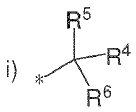 or 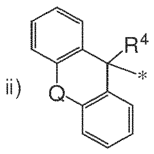
wherein:
○ R⁴ represents a hydrogen atom, a hydroxy group, a hydroxymethyl group or a linear or branched C₁₋₄ alkyl group,
○ R⁵ represents a saturated or unsaturated C₃₋₈ cycloalkyl group, a C₅₋₆ aryl group, a 5- to 6- membered heteroaryl group containing at least one heteroatom selected from , S, and O; a (C₁₋₄ alkyl)-(C₅₋₆ aryl) group, a (C₁₋₄ alkyl)-(C₃₋₈ cycloalkyl) group or a (C₁₋₄ alkyl)-(5- to 6- membered heteroaryl group containing at least one heteroatom selected from N, S, and O) group, which groups independently are optionally substituted by one or more substituents R^{a},
○ R⁶ represents a C₅₋₆ aryl group, a 5- to 6- membered heteroaryl group containing at least one heteroatom selected from N, S, and O, a saturated or unsaturated C₃₋₈ cycloalkyl group, a C₁₋₈ alkyl group, a C₂₋₈ alkenyl group, a C₂₋₈ alkynyl group, a (C₁₋₄ alkyl)-(C₅₋₆ aryl) group, a (C₁₋₄ alkyl)-(C₃₋₈ cycloalkyl) group or a (C₁₋₄ alkyl)-(5- to 6- membered heteroaryl group containing at least one heteroatom selected from N, S, and O) group, which groups independently are optionally substituted by one or more substituents R^{b},
○ R^{a} and R^{b} independently represent a halogen atom, a hydroxy group, C₁₋₄ alkyl group, C₁₋₄ alkoxy group, -SH, a C₁₋₄ alkylthio group, a nitro group, a cyano group, -CO₂R', -NR'R", -C(O)NR'R", -N(R'")C(O)-R', -N(R'")-C(O)NR'R", whererin R', R" and R"' each independently represents a hydrogen atom or a C₁₋₄ alkyl group, or R' and R" together with the nitrogen atom to which they are attached from a 3 to 6 membered heterocyclic ring.
○ Q represents a direct bond, -CH₂-, -CH₂-CH₂-, -O-, -O-CH₂-, -S-, -S-CH₂-, or -CH=CH-,
○ * represents the point of attachment of R₃ to the remainder of the molecule of formula (I),
• G is selected from the group consisting of a C₃₋₁₀ mono- or bicyclic cycloalkyl group, a C₅-C₁₄ mono- or bicyclic aryl group, a 3- to 14-membered saturated or unsaturated mono- or bicyclic heterocyclyl group having one or more heteroatoms selected from N, S and O, a 5- to 14-membered mono- or bicyclic heteroaryl group having one or more heteroatoms from N, S and O and a bicyclic ring system consisting of two monocyclic ring systems which are linked between each other by a covalent bond wherein monocyclic ring systems are independently selected from a C₃₋₈ cycloalkyl group, a C₅-C₆ aryl group, a 3- to 8-membered saturated or unsaturated heterocyclyl group having one or more heteroatoms selected from , S and O and a 5- to 6-membered heteroaryl group having one or more heteroatoms selected from N, S and O,
wherein the cyclic groups independently are optionally substituted with one or more substituents selected from a halogen atom, a C₁₋₄ alkyl group, a C₁₋₄ alkoxy group, a carboxy, group, a cyano group, a nitro group, a hydroxy group, an oxo group, a trifluoromethyl group and a trifluoromethoxy group,
with the proviso that when G is a phenyl group, L₁ is not one of the group from a direct bond, -O-, -NHC(O)-, -C(O)NH- and -NHC(O)O- group.

The invention also provides synthetic processes and intermediates described herein, which are useful for preparing compounds of the invention.

The invention further provides a pharmaceutical composition comprising at least a compound of the invention and a pharmaceutically-acceptable carrier.

The invention also provides a compound of the invention as described herein for use in the treatment of human or animal body by therapy.

The invention is also directed to the compounds as described herein, for use in the treatment of a pathological condition or disease associated with dual β2 adrenergic receptor and muscarinic receptor activities in particular wherein the pathological condition or disease is selected from a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis, preferably asthma and chronic obstructive pulmonary disease.

The invention also provides the use of the compounds of the invention as described herein, for the manufacture of a medicament for the treatment of a pathological condition or disease associated with dual β2 adrenergic receptor and muscarinic receptor activities, in particular wherein the pathological condition or disease is selected from a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis, preferably asthma and chronic obstructive pulmonary disease.

The invention is also directed to a method of treatment of a pathological condition or disease with dual β2 adrenergic receptor and muscarinic receptor activities, in particular wherein the pathological condition or disease is selected from a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis, preferably asthma and chronic obstructive pulmonary disease, comprising administering a therapeutically effective amount of the compounds of the invention or a pharmaceutical composition of the invention to a subject in need of such treatment.

The invention also provides a combination product comprising (i) at least a compound of the invention as described herein; and (ii) one or more active ingredients selected from the group consisting of a corticosteroid and/or a PDE4 inhibitor, for simultaneous, separate or sequential use in the treatment of the human or animal body.

### DETAILED DESCRIPTION OF THE INVENTION.

When describing the compounds, compositions and methods of the invention, the following terms have the following meanings, unless otherwise indicated.

As used herein the term C₁-C₄ alkyl embraces linear or branched radicals having 1 to 4 carbon atoms. Examples include methyl, ethyl, n-propyl, i-propyl, n-butyl, sec-butyl and t-butyl radicals.

As used herein, the term C₁-C₄ hydroxyalkyl embraces linear or branched alkyl radicals having 1 to 4 carbon atoms, any one of which may be substituted with one or more hydroxyl radicals.

Examples of such radicals include hydroxymethyl, hydroxyethyl, hydroxypropyl, and hydroxybutyl.

As used herein, the term C₁-C₁₀ alkylene embraces divalent alkyl moieties typically having from 1 to 10 carbon atoms. Examples of C₁-C₁₀ alkylene radicals include methylene, ethylene, propylene, butylene, pentylene and hexylene radicals.

As used herein, the term C₂-C₁₀ alkenylene embraces divalent alkenyl moieties typically having from 2 to 10 carbon atoms. Examples of C₂-C₁₀ alkenylene radicals include vinylene, propenylene, butenylene, pentenylene, hexenylene, heptenylene, octenylenyl radicals.

As used herein, the term C₂-C₁₀ alkynylene embraces divalent alkynyl moieties having 2 to 10 carbon atoms. Examples include propynylene, butynylene, heptynylene, octynylene.

As used herein, the term C₁-C₄ alkoxy (or alkyloxy) embraces optionally substituted, linear or branched oxy-containing radicals having alkyl portions of 1 to 4 carbon atoms. Exmaples include methoxy, ethoxy, n-propoxy, i-propoxy, n-butoxy, sec-butoxy and t-butoxy.

As used herein, the term C₁-C₄ alkylthio embraces radicals containing a linear or branched alkyl radicals of 1 to 4 carbon atoms attached to a divalent -S- radical. Examples include methylthio, ethylthio, n-propylthio, i-propylthio, n-butylthio, sec-butylthio and t-butylthio.

As used herein, the term C₃-C_{lO} cycloalkyl radical embraces saturated or unsaturated monocyclic carbocyclic radicals having from 3 to 10 carbon atoms. Examples of monocyclic cycloalkyl groups include cyclobutyl, cyclopentyl, cyclohexyl and cycloheptyl group.

As used herein, the term C₅-C₁₄ aryl radical embraces typically a C₅-C₁₄, preferably a C₆-C₁₄, more preferably a C₆-C₁₀ monocyclic or polycyclic aryl radical. Examples of aryl radicals include phenyl, naphthyl, naphthalenyl, anthranyl and phenanthryl.

As used herein, the term 5- to 14- membered heteroaryl radical embraces typically a 5-to 14- membered ring system comprising at least one heteroaromatic ring and containing at least one heteroatom selected from O, S and N. A 5- to 14- membered heteroaryl radical may be a single ring or two or more fused rings wherein at least one ring contains a heteroatom.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidinyl and the various pyrrolopyridyl radicals.

As used herein, the term 3- to 14-membered heterocyclyl radical embraces typically a non-aromatic, saturated or unsaturated C₃-C₁₄ carbocyclic ring system in which one or more, for example 1, 2, 3 or 4 of the carbon atoms preferably 1 or 2 of the carbon atoms are replaced by a heteroatom selected from N, O and S. A heterocyclic radical may be a single ring or two or more fused rings wherein at once ring contains a heteroatom, and may have one or more double bonds.

Examples of 3- to 14-membered heterocyclic radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, thiaranyl, aziridinyl, oxetanyl, thiatanyl, azetidinyl, 4,5-dihydro-oxazolyl, 2-benzofuran-1(3H)-one, 1,3-dioxol-2-one, tetrahydrofuranyl, 3-aza-tetrahydrofuranyl, tetrahydrothiophenyl, tetrahydropyranyl, tetrahydrothiopyranyl, 1,4-azathianyl, oxepanyl, thiephanyl, azepanyl, 1,4-dioxepanyl, 1,4-oxathiepanyl, 1,4-oxaazepanyl, 1,4-dithiepanyl, 1,4-thiezepanyl, 1,4-diazepanyl, tropanyl, (1S,5R)-3-aza-bicyclo[3.1.0]hexyl, 3,4-dihydro-2H-pyranyl, 5,6-dihydro-2H-pyranyl, 2H-pyranyl, 2,3-hydrobenzofuranyl, 1,2,3,4-tetrahydropyridinyl, 1,2,5,6-tetrahydropyridinyl, isoindolinyl and indolinyl.

As used herein, the term halogen atom embraces chlorine, fluorine, bromine or iodine atoms. A halogen atom is typically a fluorine, chlorine or bromine atom. The term halo when used as a prefix has the same meaning.

### Isomers

Compounds containing one or more chiral centre may be used in enantiomerically or diastereoisomerically pure form, in the form of racemic mixtures and in the form of mixtures enriched in one or more stereoisomer. The compounds of the invention as described and claimed encompass the racemic forms of the compounds as well as the individual enantiomers, diastereomers, and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the where the compound contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

The compounds of the present invention may exhibit the phenomena of tautomerism and structural isomerism. Tautomers exist as mixtures of a tautomeric set in solution. In solid form, usually one tautomer predominates. Even though one tautomer may be described, the present invention includes all tautomers of the compounds of the present invention.

### Polymorphs

The compounds of the present invention may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds the present invention, including all polymorphic forms ("polymorphs") thereof, are included within the scope of the invention.

### Salts

As used herein, the term pharmaceutically acceptable salt refers to a salt prepared from a base or acid which is for administration to a patient, such as a mammal. Such salts can be derived from pharmaceutically-acceptable inorganic or organic bases and from pharmaceutically-acceptable inorganic or organic acids.

As used herein, the term Pharmaceutical acceptable salt embraces salts with a Pharmaceutical acceptable acid or base. Pharmaceutically acceptable acids include both inorganic acids, for example hydrochloric, sulphuric, phosphoric, diphosphoric, hydrobromic, hydroiodic and nitric acid; and organic acids, for example citric, fumaric, gluconic, glutamic, lactic, maleic, malic, mandelic, mucic, ascorbic, oxalic, pantothenic, succinic, tartaric, benzoic, acetic, methanesulphonic, ethanesulphonic, benzenesulphonic, p-toluenesulphonic acid, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic include or primary, secondary and tertiary amines, including alkyl amines, arylalkyl amines, heterocyclyl amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, -ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred according to the invention are quaternary ammonium compounds wherein an equivalent of an anion (X⁻) is with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

### N-oxides

As used herein, an N-oxide is formed from the tertiary basic amines or imines present in the molecule, using a convenient oxidising agent.

### Isotopes

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶C fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C, are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the invention can generally be prepared by conventional techniques known to those skilled in the art or by processes analogous to those described herein, using an appropriate isatapiaally-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is present at a natural abundance of 0.015 molar %.

### Solvates

The compounds of the invention may exist in both unsolvated and solvated forms. The term solvate is used herein to describe a molecular complex comprising a compound of the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds of the present invention, such as a hydrate.

Furthermore, it is specifically contemplated that in the present invention, more than one solvent molecule may be associated with one molecule of the compounds of the present invention, such as a dihydrate. Additionally, it is specifically contemplated that in the present invention less than one solvent molecule may be associated with one molecule of the compounds of the present invention, such as a hemihydrate. Furthermore, solvates of the present invention are contemplated as solvates of compounds of the present invention that retain the biological effectiveness of the non-solvate form of the compounds.

### Prodrugs

Prodrugs of the compounds described herein are also within the scope of the invention. Thus certain derivatives of the compounds of the present invention, which derivatives may have little or no pharmacological activity themselves, when administered into or onto the body may be converted into compounds of the present invention having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

Typically G is selected from the group consisting of a C₅-C₆ aryl group, a 8- to 10-membered saturated or unsaturated bicyclic heterocyclyl group having one or more heteroatoms selected from N, S and O, a 8- to 10-membered bicyclic heteroaryl group having one or more heteroatoms selected from N, S and O and a C₅-C₆ aryl group linked to a ring system selected from a C₅-₆ aryl group, a C₃₋₇ cycloalkyl group and a 5-to 6-membered heteroaryl group having two or three heteroatoms from N, S and O, wherein the cyclic groups independently are optionally substituted with one or more substituents selected from a halogen atom, a C₁₋₄ alkyl group, a C₁₋₄ alkoxy group, a cyano group, a nitro group, a hydroxy group and an oxo group.

Preferably, G is selected from a phenyl group, a 9- to 10-membered unsaturated bicyclic heterocyclyl group having one or more heteroatoms selected from N, S and O, a 9 to 10-membered bicyclic heteroaryl group having one or more heteroatoms selected from N, S and O and a C₅-C₆ aryl group linked to a ring system selected from a C₅₋₆ aryl group and a 5- to 6-membered heteroaryl group having two or three heteroatoms selected from N, S and O, wherein the cyclic groups independently are optionally substituted with one or two substituents selected from a halogen atom, a methyl group, a methoxy group, a cyano group, a hydroxy group and an oxo group.

Typically, L₁ is selected from the group consisting of direct bond, -NR^{c}-, -S-, -SO₂-, - C(O)-, -C(O)O-, -S(O)₂NR^{c}-, -NR^{c}S(O)₂-, -NR^{c}(CO)(CH₂)O-, -O(CH₂)(CO)NR^{c}-, - NR^{c}(CO)NR^{d}- and -CONR^{c}S(O)₂-, wherein R^{c} and R^{d} independently are selected from a hydrogen atom and a methyl group.

Preferably L₁ is selected from a direct bond, -NH-, -S-, -SO₂-, -C(O)-, -NR^{c}(CO)NR^{c}-and -O(CH₂)(CO)NR^{c}-; more preferably L₁ is selected from a direct bond, -NH-, -SO₂-, -NH(CO)NH- and -O(CH₂)(CO)NR^{c}-; being most preferably a direct bond or - O(CH₂)(CO)NR^{c}-.

In a preferred embodiment of the present invention, -G-L₁- has the following formula: 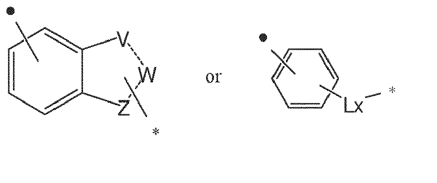 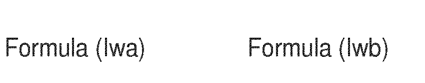 wherein
- V, W and Z are independently selected from a -N-, -C-, -S-, -O- and -C(O)-
- Lx represents a 5- to 6- membered heteroaryl group having at least one heteroatom selected from N, S and O, or Lx represents a -O-CH₂-CO-NR^{d}-, wherein R^{d} represents a hydrogen atom or a methyl group,
- * represents the point of attachment with A₂ and
- represents the point of attachment with A₁.

In a preferred embodiment of the present invention, -G-L₁- has the following formula (Iwa): 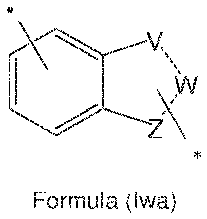 wherein V, W and Z are as defined above.

In a still preferred embodiment,
- Z is a nitrogen atom,
- V represents a nitrogen atom, an oxigen atom, a carbon atom or a sulphur atom and,
- W represents a nitrogen atom, a carbon atom or a carbonyl atom.

More preferably, -G-L₁- has the following formula (Iwaa): 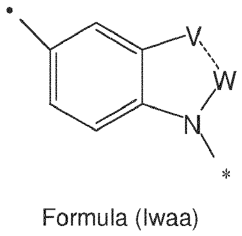 wherein V and W are as defined above.

Typically R³ represents a group of formula: 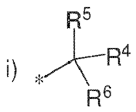 or 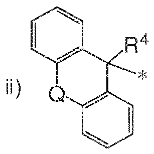 wherein:
○ R⁴ represents a hydrogen atom, a hydroxy group, a hydroxymethyl group or a linear or branched C₁₋₄ alkyl group,
○ R⁵ and R⁶ independently represent C₅₋₆ aryl group, a 5- to 6- membered heteroaryl group containing at least one heteroatom selected from N, S, and O; (C₁₋₄ alkyl)-(C₅₋₆ aryl) group, a C₃₋₈ cycloalkyl group,
○ Q represents a direct bond or a -CH₂-, -CH₂-CH₂-, -O-, -O-CH₂-, -S-, -S-CH₂-,or-CH=CH-,
○ * represents the point of attachment of R₃ to the remainder of the molecule of formula (I),

More preferably R³ represents a group of formula i) or ii), wherein:
○ R⁴ represents a hydrogen atom, a hydroxy group, a hydroxymethyl group or a linear or branched C₁₋₄ alkyl group,
○ R⁵ and R⁶ independently represents a thienyl group, a phenyl group, a benzyl group or a C₄₋₆ cycloalkyl group,
○ Q represents a direct bond or an oxygen atom,
○ * represents the point of attachment of R₃ to the remainder of the molecule of formula (I),

In another embodiment, compounds of the present invention have the following formula (IA): 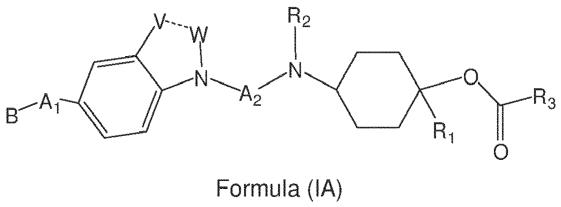 wherein R₁, R₂, R₃, A₁, A₂, V, W and B are as defined above.

Typically, A₁ and A₂ independently are selected from the group consisting of C₁₋₆ alkylene group, C₁₋₆ alkenylene group and C₁₋₆ alkynylene group, wherein said groups are optionally substituted with one or more substituents selected from a halogen atom, a hydroxy group, a C₁₋₂ alkyl group, a C₁₋₂ alkoxy group, a C₅₋₆ aryl group and a C₃₋₆ cycloalkyl group.

Preferably, A₁ and A₂ independently represent a C₁₋₆ alkylene group optionally substituted with one or more substituents selected from a C₁₋₂ alkyl group, a C₁₋₂ alkoxy group and a phenyl group, preferably substituted with one or two substituents selected from a methyl group and a methoxy group, more preferably a methyl group.

Typically B represents a group of formula (IB): 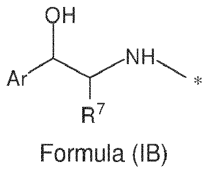 wherein:
- R⁷ is selected from the group consisting of a hydrogen atom, a linear or branched C₁₋₄ alkyl group and a linear or branched C₁₋₄ alkoxy group,
- Ar is selected from the group consisting of a C₃₋₁₀ saturated or unsaturated, mono- or bicyclic cycloalkyl group, a C₅-C₁₄ mono- or bicyclic aryl group, a 3 to 14-membered saturated or unsaturated mono- or bicyclic heterocyclyl group having one or more heteroatoms selected from N, S and O, a 5- to 14-membered mono- or bicyclic heteroaryl group having one or more heteroatoms selected from N, S and O and wherein the cyclic groups independently are optionally substituted with one or more substituents selected from a halogen atom, a cyano group, a nitro group, an oxo group, a carboxy group, a C₁₋₄ alkyl group, a C₁₋₄ alkoxy group, -CF₃, -OCF₃, -NR^{e}R^{f}, -(CH₂)ₚ-OH, -NR^{e}(CO)R^{f}, - NR^{e}-SO₂-R^{g}, -SO₂NR^{e}R^{f}, -OC(O)R^{h}, and -NR^{e}(CH₂)₍₀₋₂₎-Rⁱ, wherein p has a value of 0, 1 or 2 and wherein:
   R^{e} and R^{f} independently represent a hydrogen atom or a linear or branched C₁₋₄ alkyl group,
   R^{g} is selected from the group consisting of a linear or branched C₁₋₄ alkyl group, a C₆₋₅ aryl group, a saturated or unsaturated C₃₋₈ cycloalkyl, wherein the cyclic groups independently are optionally substituted with one or more substituents selected from a halogen atom, a C₁₋₄ alkyl group and a C₁₋₄ alkoxy group, R^{h} is selected from a hydrogen atom.-NR^{e}R^{f} and a C₅₋₆ aryl group which is optionally substituted with one or more substituents selected from a C₁₋₄ alkyl group and a C₁₋₄ alkoxy group,
   Rⁱ is selected from the group consisting of a C₅₋₆ aryl group, a C₃₋₈ cycloalkyl group and a 3 to 8 membered saturated or unsaturated heterocyclyl group, which groups independently are optionally substituted with one or more substituents selected from a halogen atom, a C₁₋₄ alkyl group and a C₁₋₄ alkoxy group.

Preferably, Ar represents a group of formula: 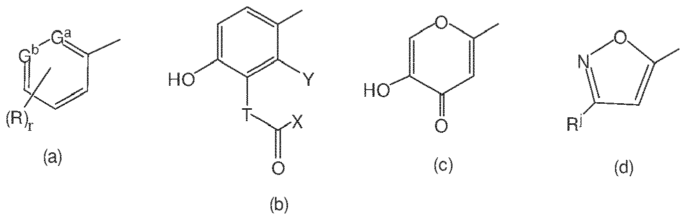 wherein
- G^{a} and G^{b} independently are selected from a nitrogen atom and a carbon atom,
- r has a value of 0, 1, 2 or 3 and
- R is seleted from the group consisting of a halogen atom, an amino group, a cyano group, a nitro group, an oxo group, a carboxy group, a C₁₋₄ alkyl group, a C₁₋₄ alkoxy group, -CF₃, -OCF₃, -(CH₂)ₚ-OH, -NH(CO)H, -NH-SO₂-R^{g}, -SO₂NH₂, -OC(O)H, -O(CO)-(4-methyl)phenyl, -O(CO)-N(CH₃)₂, -OC(O)NH₂ and - NH(CH₂)₍₁₋₂₎-Rⁱ, group, wherein p is as defined above and R^{g} and Rⁱ independently are selected from a phenyl group optionally substituted with a one substituent selected from methyl group or a methoxy group,
- R^{j} represents a halogen atom,
- T is selected from the group consisting of -CH₂- and -NH-,
- Both X and Y represent a hydrogen atom or X together with Y form the group - CH₂-CH₂-, -CH=CH-, -CH₂-O- or -S-, wherein in the case of -CH₂-O- the methylene group is bound to the carbonyl group holding X and the oxygen atom is bound to the carbon atom in the phenyl ring holding Y,

Preferably, Ar represents a compound of formula (a) or (b) wherein: 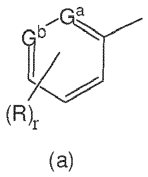 or 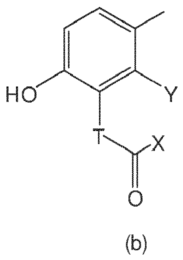
- Both G^{a} and G^{b} represent a carbon atom,
- R is seleted from the group consisting of halogen atom, amino group, cyano group, nitro group, -(CH₂)ₚ-OH, -NH(CO)H, -NH-SO₂-CH₃, -SO₂NH₂, -OC(O)H, -O(CO)-(4-methyl)phenyl, -O(CO)-N(CH₃)₂, -OC(O)NH₂ and -CF₃ group, wherein p has a value of 0, 1 or 2,
- T represents -NH- group,
- Both X and Y represent a hydrogen atom or X together with Y form the group - CH=CH-, -CH₂-CH₂-, -CH₂-O- or -S-, wherein in the case of -CH₂-O- the methylene group is bound to the carbon atom in the amido substituent holding X and the oxygen atom is bound to the carbon atom in the phenyl ring holding Y

In a still prefered embodiment Ar is selected from the group consisting of 3-bromoisoxazol-5-yl, 3,4-dihydroxyphenyl, 4-hydroxy-3-(methylsulfonamido)phenyl, 3,4-bis(4-methylbenzoyloxy)phenyl, 3,5-bis(dimethylcarbamoyloxy)phenyl, (5-hydroxy-6-hydroxymethyl)pyrid-2-yl, (4-amino-3,5-dichloro)phenyl, 4-hydroxyphenyl, 4-hydroxy-3-(2-hydroxyethyl)phenyl, 4-hydroxy-3-(hydroxymethyl)phenyl, [4-amino-3-chloro-5-(trifluoromethyl)]phenyl, (3-formamido-4-hydroxy)phenyl, 8-hydroxy-2-oxo-1,2-dihydroquinolin-5-yl, 8-hydroxy-2-oxo-1,2,3,4-tetrahydroquinolin-5-yl, 5-hydroxy-3-oxo-3,4-dihydro-2*H*-benzo[*b*][1,4]oxazin-8-yl, 4-hydroxy-2-oxo-2,3-dihydrobenzo[d]thiazol-7-yl. Preferably Ar is selected from the group consisting of 4-hydroxy-3-(hydroxymethyl)phenyl , (3-formamido-4-hydroxy)phenyl, 8-hydroxy-2-oxo-1,2-dihydroquinolin-5-yl, 8-hydroxy-2-oxo-1,2,3,4-tetrahydroquinolin-5-yl and 5-hydroxy-3-oxo-3,4-dihydro-2*H*-benzo[*b*][1,4]oxazin-8-yl.

In another embodiment Ar represents a compound of formula (b) wherein X and Y are as defined above and T represents a -NH- group.

Still in another embodiment of the present invention, compounds of the present invention have the following formula (IC):

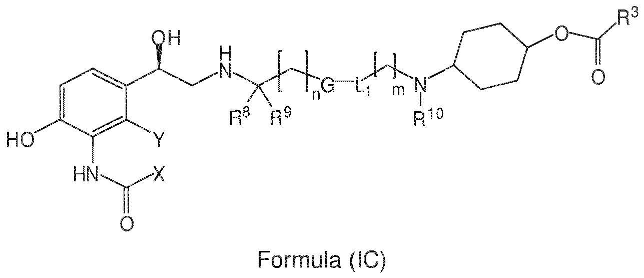

Wherein:
- R³ represents a group of formula: 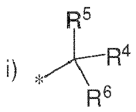 or 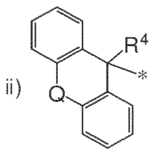 wherein:
   ○ R⁴ represents a hydrogen atom, a hydroxy group, a hydroxymethyl group or a linear or branched C₁₋₄ alkyl group,
   ○ R⁵ and R⁶ independently represent C₅₋₆ aryl group, a 5- to 6- membered heteroaryl group containing at least one heteroatom selected from N, S, and O; (C₁₋₄ alkyl)-(C₅₋₆ aryl) group, a C₃₋₈ cycloalkyl group,
   ○ Q represents a direct bond or a -CH₂-, -CH₂-CH₂-, -O-, -O-CH₂-, -S-, -S-CH₂-,or-CH=CH-,
   ○ * represents the point of attachment of R₃ to the remainder of the molecule of formula (I),
- Both X and Y represent a hydrogen atom or X together with Y form the group - CH=CH-, -CH₂-CH₂-, -CH₂-O- or -S-, wherein in the case of -CH₂-O- the methylene group is bound to the carbon atom in the amido substituent holding X and the oxygen atom is bound to the carbon atom in the phenyl ring holding Y
- n has a value of 0, 1 or 2,
- m has a value of 2, 3 or 4,
- R⁸, R⁹ and R¹⁰ independently represent a hydrogen atom or a linear or branched C₁₋₄ alkyl group,
- -G-L₁- represents a group of formula: 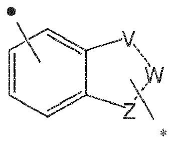 or 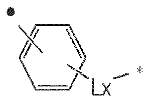 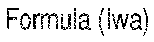 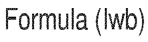
wherein
- V, W and Z are independently selected from a -N-, -C-, -S-, -O- and -C(O)-,
- Lx represents a 5- to 6- membered heteroaryl group having at least one heteroatom selected from , S and O, or Lx represents a -O-CH₂-CO-NR^{d}-, wherein R^{d} represents a hydrogen atom or a methyl group,
- * represents the point of attachment with the moiety containing the cyclohexyl group and
- represents the point of attachment with the moiety containing the aminoethylphenol moiety.

In a still preferred embodiment, Lx represents a 5 to 6 membered heteroaryl group having at least one heteroatom selected from N, S and O. Preferably Lx is selected from a pyridyl, a pyrazinyl, a furyl, an oxadiazolyl, a imidazolyl, a thiazolyl and a thienyl group, more preferably, Lx represents a pyridyl, an oxadiazolyl, a imidazolyl or a thiazolyl group, being most preferably an oxadiazolyl group.

In a preferred embodiment, compounds of the present invención have the following formula (IDa): 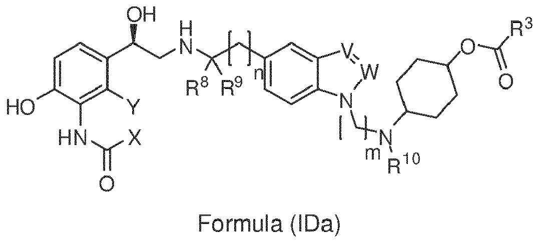 wherein V, W, X, Y, R⁸, R⁹, R¹⁰, n and m are as defined above.

In a still preferred embodiment, compounds of the present invention have the following formula (ID):

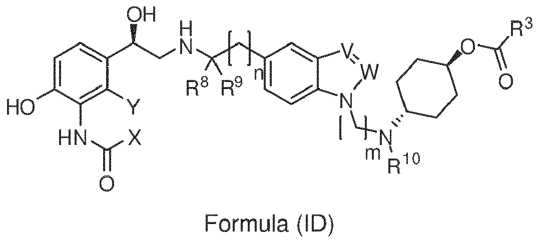

Wherein:
- V, W, X, Y, R⁸ R⁹, R¹⁰, n and m are as defined above,
- R³ represents a group of formula: 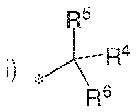 or 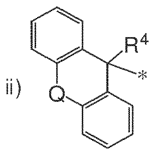 wherein:
   ○ R⁴ represents a hydrogen atom, a hydroxy group, a hydroxymethyl group or a linear or branched C₁₋₄ alkyl group,
   ○ R⁵ and R⁶ independently represents a thienyl group, a phenyl group, a benzyl group or a C₄₋₆ cycloalkyl group,
   ○ Q represents a direct bond or an oxygen atom,
   ○ * represents the point of attachment of R₃ to the remainder of the molecule of formula (I),

Typically, X together with Y form the group -CH=CH- or -CH₂-CO-. Preferably, X together with Y form the group -CH=CH-.

Typically W represents a nitrogen atom or a carbonyl group, preferably W represents a nitrogen atom.

Typically, V represents a nitrogen atom, an oxygen atom or a sulphur atom, preferably V is a nitrogen atom or an oxygen atom.

In a preferred embodiment V represents a nitrogen atom or an oxygen atom while W represents a carbonyl group.

In another preferred embodiment both V and W represent a nitrogen atom.

Typicall, n has a value 0.

Typically, m has a value of 3.

Typically R¹⁰ represents a hydrogen atom or a methyl group, preferably a methyl group.

Typically, R⁸ and R⁹ independently represent a hygrogen atom or a methyl group, preferably both R⁸ and R⁹ represent a hydrogen atom.

Typically, R₃ represents a group of formula ii), wherein Q is an oxygen atom and R⁴ is selected from a hydrogen atom, a hydroxy group and a methyl group. Preferably R⁴ represents a hydroxy group or a methyl group, more preferably a methyl group.

Typically, R₃ represents a group of formula i) wherein:
- R⁴ represents a hydrogen atom, a methyl group or a hydroxy group, preferably R^{a} represents a hydroxy group,
- R⁵ and R⁶ independently represent a thienyl group, a cyclopentyl group or a benzyl group, preferably both R⁵ and R⁶are thienyl groups.

In one embodiment of the present invention, in compounds of formula (IC)
- -G-L₁- represents a group of formula: 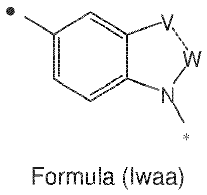 or 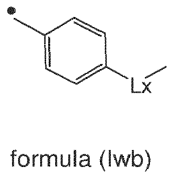 Wherein
   ○ V is selected from -N-, -C-, -S- and -O-,
   ○ W is selected from -N-, -C-, and -C(O)-,
   ○ Lx represents an oxadiazolyl group or -O-CH₂-CO-NR^{d}, wherein R^{d} represents a hydrogen atom or a methyl group.
   ○ * represents the point of attachment with the moiety containing the cyclohexyl group and
   ○ • represents the point of attachment with the moiety containing the aminoethylphenol fragment,
- R⁸ and R⁹ independently are selected from a hydrogen atom and a methyl group,
- R¹⁰ represents a methyl group,
- n has a value of 0 or 1,
- m has a value of 2, 3 or 4,
- Both X and Y represents a hydrogen atom or X together with Y form -CH=CH-, -CH₂-O-, or-S-group,
- R₃ represents a group of formula: 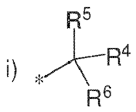 or 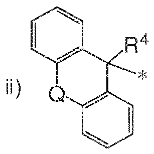 wherein:
   ○ R⁴ represents a methyl group or a hydroxy group,
   ○ R⁵ and R⁶ independently represents a thienyl group, a phenyl group, benzyl group or a cyclopentyl group,
   ○ Q represents a direct bond or an oxygen atom,
   ○ * represents the point of attachment of R³ to the remainder of the molecule of formula (I).

Preferably,
- -G-L₁- represents a group of formula: 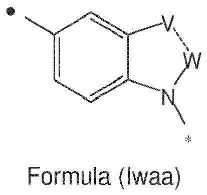 Wherein
   o W represents a nitrogen atom or a carbonyl group,
   o V represents a nitrogen or an oxygen atom,
- Both R⁸ and R⁹ represents a hydrogen atom,
- X together with Y form -CH=CH-,
- R₃ represents a group of formula i) wherein R⁴ represents a hydroxy group and both R⁵ and R⁶ represent a thienyl group.

Particular individual compounds of the invention include:
Trans-4-[{3-(6-({[(2R)-2-hydroxy-2-(8-hydroxy-2-oxo-1,2-dihydroquinolin-5-yl)ethyl] amino}methyl)-2-oxo-1,3-benzoxazol-3(2H)-yl]propyl}(methyl)amino]-cyclohexyl hydroxy(di-2-thienyl)acetate, dihydrofluoride,
trans-4-[{3-(5-({[(2R)-2-hydroxy-2-(8-hydroxy-2-oxo-1,2-dihydroquinolin-5-yl)ethyl]amino}methyl)-2-oxo-2,3-dihydro-1H-benzimidazol-1-yl]propyl} (methyl)amino]cyclohexyl hydroxy(di-2-thienyl)acetate dihydrofluoride, Trans-4-[{3-(5-({[(2R)-2-hydroxy-2-(8-hydroxy-2-oxo-1,2-dihydroquinolin-5-yl)ethyl]amino}methyl)-1H-1,2,3-benzotriazol-1-yl]propyl}(methyl)amino] cyclohexylhydroxy(di-2-thienyl)acetate dihydrofluoride,
Trans-4-[{3-[5-({[(2R)-2-hydroxy-2-(8-hydroxy-2-oxo-1,2-dihydroquinolin-5-yl)ethyl]amino}methyl)-1-indol-1-yl]propyl}(methyl)amino]cyclohexylhydroxy (di-2-thienyl)acetate dihydrofluoride,
Trans-4-[{3-(5-({[(2R)-2-hydroxy-2-(8-hydroxy-2-oxo-1,2-dihydroquinolin-5-yl)ethyl]amino}methyl)-1H-benzimidazol-1-yl]propyl}(methyl)amino]cyclohexyl hydroxy(di-2-thienyl)acetate,
Trans-4-[{3-[5-({[(2R)-2-hydroxy-2-(8-hydroxy-2-oxo-1,2-dihydroquinolin-5-yl) ethyl]amino}methyl)-1H-indazol-1-yl]propyl}(methyl)amino]cyclohexylhydroxy(di-2-thienyl)acetate,
Trans-4-[{3-[6-({[(2R)-2-hydroxy-2-(8-hydroxy-2-oxo-1,2-dihydroquinolin-5-yl)ethyl]amino}methyl)-2-oxo-1,3-benzothiazol-3(2H)-yl]propyl}(methyl)amino] cyclohexyl hydroxy(di-2-thienyl)acetate,
Trans-4-[(3-{5-[({1(2R)-2-[3-(formylamino)-4-hydroxyphenyl]-2-hydroxyethyl}amino)methyl]-1H-1,2,3-benzotriazol-1-yl}propyl)(methyl)amino] cyclohexyl hydroxy(di-2-thienyl)acetate,
Trans-4-[{3-[5-({[(2R)-2-hydroxy-2-(4-hydroxy-2-oxo-2,3-dihydro-1,3-benzothiazol-7-yl)ethyl]amino}methyl)-1H-1,2,3-benzotriazol-1-yl]propyl}(methyl) amino]cyclohexyl hydroxy(di-2-thienyl)acetate,
Trans-4-[{3-[6-(2-{[(2R)-2-hydroxy-2-(8-hydroxy-2-methylene-1,2-dihydroquinolin-5-yl)ethyl]amino}ethyl)-2-oxo-1,3-benzoxazol-3(2H)-yl]propyl}(methyl)amino] cyclohexyl hydroxy(di-2-thienyl)acetate,
Trans-4-[12-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]ethyl}(methyl)amino] cyclohexylhydroxy(di-2-thienyl)acetate,
Trans-4-[{4-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]butyl}(methyl)amino] cyclohexylhydroxy(di-2-thienyl)acetate,
Trans-4-[{3-[5-({[(2R)-2-hydroxy-2-(8-hydroxy-2-oxo-1,2-dihydroquinolin-5-yl)ethyl]amino}methyl)-1H-1,2,3-benzotriazol-1-yl]propyl}(methyl)amino] cyclohexylcyclopentyl(hydroxy)2-thienylacetate,
Trans-4-[{3-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]propyl}(methyl)amino] cyclohexyl-9-methyl-9H-xanthene-9-carboxylate,
Trans-4-[{3-[6-({[(2R)-2-hydroxy-2-(5-hydroxy-3-oxo-3,4-dihydro-2H-1,4-benzoxazin-8-yl)ethyl]amino}methyl)-2-oxo-1,3-benzothiazol-3(2H)-yl]propyl} (methyl)amino]cyclohexyl hydroxy(di-2-thienyl)acetate,
Trans-4-[(2-{5-[({(2R)-2-[3-(formylamino)-4-hydroxyphenyl]-2-hydroxyethyl} amino)methyl]-1H-indol-1-yl}ethyl)(methyl)amino]cyclohexyl 9H-fluorene-9-carboxylate,
Trans-4-[(3-{5-[({(2R)-2-[3-(formylamino)-4-hydroxyphenyl]-2-hydroxyethyl} amino)methyl]-1H-indol-1-yl}propyl)(methyl)amino]cyclohexyl 2-hydroxy-3-phenyl-2-(2-thienyl)propanoate,
Trans-4-[{3-[5-(2-{[(2R)-2-hydroxy-2-(8-hydroxy-2-oxo-1,2-dihydroquinolin-5-yl)ethyl]amino}-2-methylpropyl)-2-oxo-2,3-dihydro-1H-benzimidazol-1-yl]propyl} (methyl)amino]cyclohexyl 2,2-diphenylpropanoate,
Trans-4-[{2-[5-(2-{[(2R)-2-hydroxy-2-(8-hydroxy-2-oxo-1,2-dihydroquinolin-5-yl) ethyl]amino}-2-methylpropyl)-1H-indazol-1-yl]ethyl}(methyl)amino]cyclohexyl2-phenyl-2-(2-thienyl)propanoate,
Trans-4-[{3-[6-{2-{[(2R)-2-hydroxy-2-(8-hydroxy-2-methylene-1,2-dihydroquinolin-5-yl)ethyl]amino}propyl)-2-oxo-1,3-benzoxazol-3(2H)-yl]propyl}(methyl)amino] cyclohexyl hydroxy(di-2-thienyl)acetate,
Trans-4-[(3-{3-[4-({[(2R)-2-hydroxy-2-(8-hydroxy-2-oxo-1,2-dihydroquinolin-5-yl)ethyl]amino}methyl)phenyl]-1,2,4-oxadiazol-5-yl}propyl)(methyl)amino]cyclohexyl hydroxy(di-2-thienyl)acetate,
Trans-4-[{2-[{[4-({[(2R)-2-hydroxy-2-(8-hydroxy-2-oxo-1,2-dihydroquinolin-5-yl)ethyl]amino}methyl)phenoxy]acetyl}(methyl)amino]ethyl}(methyl)amino]cyclohexy I hydroxy(di-2-thienyl)acetate,
Trans-4-[[2-({(4-({[(2R)-2-hydroxy-2-(8-hydroxy-2-oxo-1,2-dihydroquinolin-5-yl)ethyl]amino}methyl)phenoxy]acetyl}amino)ethyl](methyl)amino]cyclohexyl hydroxy(di-2-thienyl)acetate,
Trans-4-[(3-{3-[2-chloro-4-({[(2R)-2-hydroxy-2-(8-hydroxy-2-oxo-1,2-dihydroquinolin-5-yl)ethyl]amino}methyl)-5-methoxyphenyl]-1,2,4-oxadiazol-5-yl}propyl)(methyl)amino]cyclohexyl hydroxy(di-2-thienyl)acetate and
Trans-4-[{2-[{[2-chloro-4-({[(2R)-2-hydroxy-2-(8-hydroxy-2-oxo-1,2-dihydroquinolin-5-yl)ethyl]amino}methyl)-5-methoxyphenoxy]acetyl}(methyl)amino]ethyl}(methyl) amino]cyclohexyl hydroxy(di-2-thienyl)acetate,
or pharmaceutically acceptable salts or N-oxides or solvates or deuterated derivative thereof:

Of particular interest are the compounds:
Trans-4-[{3-[6-({[(2R)-2-hydroxy-2-(8-hydroxy-2-oxo-1,2-dihydroquinolin-5-yl)ethyl] amino}methyl)-2-oxo-1,3-benzoxazol-3(2H)-yl]propyl}{methyl)amino]-cyolohexyl hydroxy(di-2-thienyl)acetate, dihydrofluoride,
trans-4-[{3-[5-({[(2R)-2-hydroxy-2-{8-hydroxy-2-oxo-1,2-dihydroquinolin-5-yl)ethyl]amino}methyl)-2-oxo-2,3-dihydro-1-benzimidazol-1-yl]propyl} (methyl)amino]cyclohexyl hydroxy(di-2-thienyl)acetate dihydrofluoride, Trans-4-[{3-[5-({[(2R)-2-hydroxy-2-(8-hydroxy-2-oxo-1,2-dihydroquinolin-5-yl)ethyl]amino}methyl)-1H-1,2,3-benzotriazol-1-yl]propyl}(methyl)amino] cyolohexylhydroxy(di-2-thienyl)acetate dihydrofluoride,
Trans-4-[{3-[5-({[(2R)-2-hydroxy-2-(8-hydroxy-2-oxo-1,2-dihydroquinolin-5-yl)ethyl]amino}methyl)-1H-indol-1-yl]propyl}(methyl)amino]cyclohexylhydroxy (di-2-thienyl)acetate dihydrofluoride,
Trans-4-[{3-[6-({[(2R)-2-hydroxy-2-(8-hydroxy-2-oxo-1,2-dihydroquinolin-5-yl)ethyl]amino}methyl)-2-oxo-1,3-benzothiazol-3(2H)-yl]propyl}(methyl)amino] cyclohexyl hydroxy(di-2-thienyl)acetate,
Trans-4-[(3-{5-[({(2R)-2-[3-(formylamino)-4-hydroxyphenyl]- 2-hydroxyethyl}amino)methyl]-1H-1,2,3-benzotriazol-1-yl}propyl)(methyl)amino] cyclohexyl hydroxy(di-2-thienyl)acetate,
Trans-4-[{3-[5-({[(2R)-2-hydroxy-2-(4-hydroxy-2-oxo-2,3-dihydro-1,3-benzothiazol-7-yl)ethyl]amino}methyl)-1H-1,2,3-benzotriazol-1-yl]propyl}(methyl) amino]cyclohexyl hydroxy(di-2-thienyl)acetate,
Trans-4-[{2-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]ethyl}(methyl)amino] cyclohexylhydroxy(di-2-thienyl)acetate,
Trans-4-[{3-[5-({[(2R)-2-hydroxy-2-(8-hydroxy-2-oxo-1,2-dihydroquinolin-5-yl)ethyl]amino}methyl)-1H-1,2,3-benzotriazol-1-yl]propyl}(methyl)amino] cyclohexylcyclopentyl(hydroxy)2-thienylacetate,
Trans-4-[{3-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]propyl}(methyl)amino] cyclohexyl-9-methyl-9H-xanthene-9-carboxylate,
Trans-4-[{2-[{[4-({[(2R)-2-hydroxy-2-(8-hydroxy-2-oxo-1,2-dihydroquinolin-5-yl)ethyl] amino}methyl)phenoxy]acetyl}(methyl)amino]ethyl}(methyl)amino]cyclohexyl hydroxy(di-2-thienyl)acetate,
Trans-4-[[2-({[4-({[(2R)-2-hydroxy-2-(8-hydroxy-2-oxo-1,2-dihydroquinolin-5-yl)ethyl] amino}methyl)phenoxy]acetyl}amino)ethyl](methyl)amino]cyclohexyl hydroxy(di-2-thienyl)acetate, and
Trans-4-[(3-{3-[2-chloro-4-({[(2R)-2-hydroxy-2-(8-hydroxy-2-oxo-1,2-dihydroquinolin-5-yl)ethyl]amino}methyl)-5-methoxyphenyl]-1,2,4-oxadiazol-5-yl}propyl) (methyl)amino]cyclohexyl hydroxy(di-2-thienyl)acetate,
or Pharmaceutical acceptable salts or N-oxides or solvates or deuterated derivative thereof:

The invention is also directed to a compound of the invention as described herein for use in the treatment of the human or animal body by therapy.

According to another embodiment the present invention covers pharmaceutical compositions comprising at least a compound of the ivenntion, as hereinabove described, in admixture with pharmaceutically acceptable diluents or carriers.

In an embodiment of the present invention the pharmaceutical composition further comprises a therapeutically effective amount of one or more other therapeutic agents, in particular one or more drugs selected from the group consisting of corticosteroids, and PDE4 inhibitors.

It is also an embodiment of the present invention that the pharmaceutical composition is formulated for administration by inhalation.

The compounds of the present invention as hereinabove defined may also be combined with one or more other therapeutic agents, in particular one or more drugs selected from the group consisting of corticosteroids and PDE4 inhibitors, for simultaneous, separate or sequential use in the treatment of the human or animal body.

The invention is also directed to compounds of the present invention for use in the treatment of a pathological condition or disease associated with both β2 adrenergic receptor and muscarinic receptor activities such as a pulmonary disease. In particular the pulmonary disease is asthma or chronic obstructive pulmonary disease.

The pathological condition or disease can also be applied within the scope of the present invention to the treatment of a disease or condition selected from the group consisting of pre-term labor, glaucoma, neurological disorders, cardiac disorders, and inflammation, urological disorders such as urinary incontinence and gastrointestinal disorders such as irritable bowel syndrome or spastic colitis.

The invention is also directed to the use of compounds of the present invention for the manufacture of a medicament for the treatment of pathological condition or disease associated with one or both β2 adrenergic receptor and muscarinic receptor activities such as a pulmonary disease, in particular asthma or chronic obstructive pulmonary disease, pre-term labor, glaucoma, neurological disorders, cardiac disorders, inflammation, urological disorders and gastrointestinal disorders, preferably, asthma and chronic obstructive pulmonary disease.

The invention is also directed to a method of treating these diseases, which comprises administering a therapeutically effective amount of a pharmaceutical composition comprising a dual β2 adrenergic receptor agonists and muscarinic receptor antagonists according to the present invention. The method further comprises administering a therapeutically effective amount of one or more other therapeutic agent selected from the group consisting of a corticosteroid and a PDE4 inhibitor.

The invention is also directed to a method of modulating the activity of a β2 adrenergic and/or a M3 receptor, the method comprising stimulating a β2 adrenergic receptor and/or blocking a M3 receptor with a modulatory amount of compounds of the present invention.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with β2 adrenergic receptor and muscarinic activities" includes all disease states and/or conditions that are acknowledged now, or that are found in the future, to be associated with both β2 adrenergic receptor and muscarinic receptor activity. Such disease states include, but are not limited to, pulmonary diseases, such as asthma and chronic obstructive pulmonary disease (including chronic bronchitis and emphysema), as well as neurological disorders and cardiac disorders. β2 adrenergic receptor activity is also known to be associated with pre-term labor (see International Patent Application Publication Number WO 98/09632), glaucoma and some types of inflammation (see International Patent Application Publication Number WO 99/30703 and Patent Application Publication Number EP 1 078 629).

On the other hand M3 receptor activity is associated with gastrointestinal-tract disorders such as Irritable bowel syndrome (IBS) (see, for ex., US5397800), GI ulcers , spastic colitis (see, for ex., US 4556653); urinary-tract disorders such as urinary incontinence (see, for ex., J.Med.Chem., 2005, 48, 6597-6606), pollakiuria; motion sickness and vagally induced sinus bradycardia.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

The term "amino-protecting group" refers to a protecting group suitable for preventing undesired reactions at amino nitrogen. Representative amino-protecting groups include, but are not limited to, formyl; acyl groups, for example alkanoyl groups such as acetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl; silyl groups such as trimethylsilyl (TMS) and tert-butyldimethylsilyl (TBS); and the like.

The term "hydroxy-protecting group" refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl; arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

Processes for preparing compounds of the invention are provided as further embodiments of the invention and are illustrated by the procedures below.

One of the most convenient synthetic way for the preparation of compounds of formula (ID) is depicted in Scheme 1.

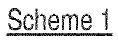

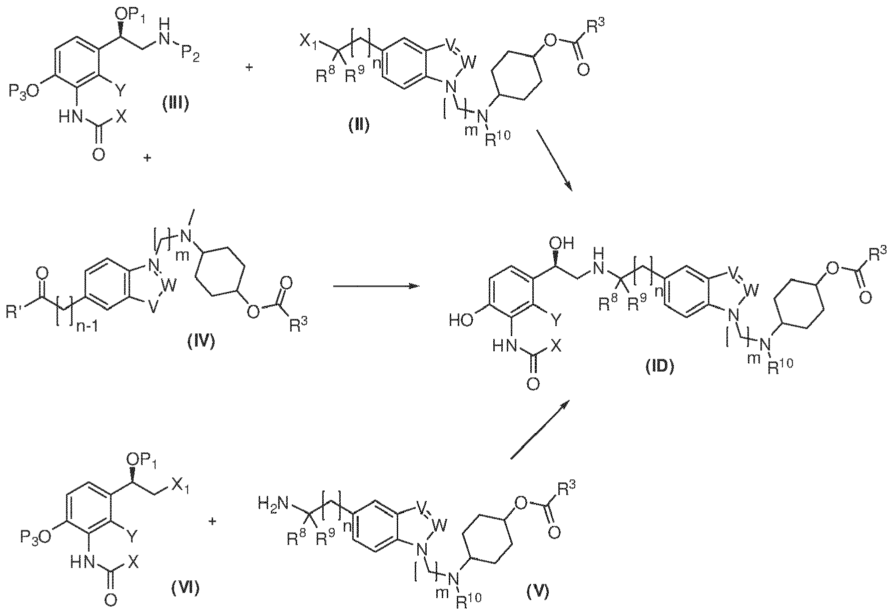

Compounds of formula **(ID)** may be prepared by reacting Intermediates of formula **(II),** wherein X₁ represents a leaving group such as a halogen atom or an active ester as mesylate or tosylate, with intermediates of formula **(III),** wherein P₁ and P₃ independently represent a hydrogen atom or an oxygen-protecting group such as a silyl or benzyl ether and P₂ represents a hydrogen atom or a nitrogen-protecting group such as for example a benzyl group. This reaction is best carried out in an aprotic polar solvent such as dimethylformamide (DMF), 1-methyl-2-pyrrolidone or dimethtylsulfoxie (DMSO) in a range of temperatures between room temperature and 200ºC, in the presence of an acid scavenger such as sodium hydrogen carbonate or a tertiary amine.

Alternatively, compounds of formula **(ID)** may be prepared by reacting intermediates of formula **(V)** with intermediates of formula **(VI)** wherein X₁, P₁ and P₃ have the same meaning as disclosed above, following the same synthetic procedure disclosed above; and subsequently removing whichever protecting group present in the intermediate to provide a compound of formula **(ID).** Such deprotection processes involve, for example, a desilylation process, by using triethylamine trihydrofluoride, TBAF, hydrogen chloride or other acidic reagents in an inert solvent like THF in a range of temperatures between 0ºC and 50ºC. The deprotection could also be carried out by a debenzylation process, for example, by hydrogenating the compound in the presence of a catalyst such as palladium on charcoal in an inert solvent like ethanol or THF or a mixture of solvents. This reaction is typically carried out at a hydrogen pressure between 10 and 60 psi and in a range of temperatures between room temperature and 50ºC.

In another alternative way, compounds of formula **(ID)** with R⁹ = H may also be prepared by reacting intermediates of formula **(IV)** with intermediates of formula **(III).** This reaction is best carried out in a solvent or mixture of solvents like THF, methanol, dichloromethane or DMSO at a temperature between 0ºC and 60ºC using a hydride like sodium borohydride or sodium triacetoxyborohydride as reducing agent.

Intermediates of formula **(II)** may be prepared from commercially available starting materials and reagents using well known procedures, as depicted in Scheme 2.

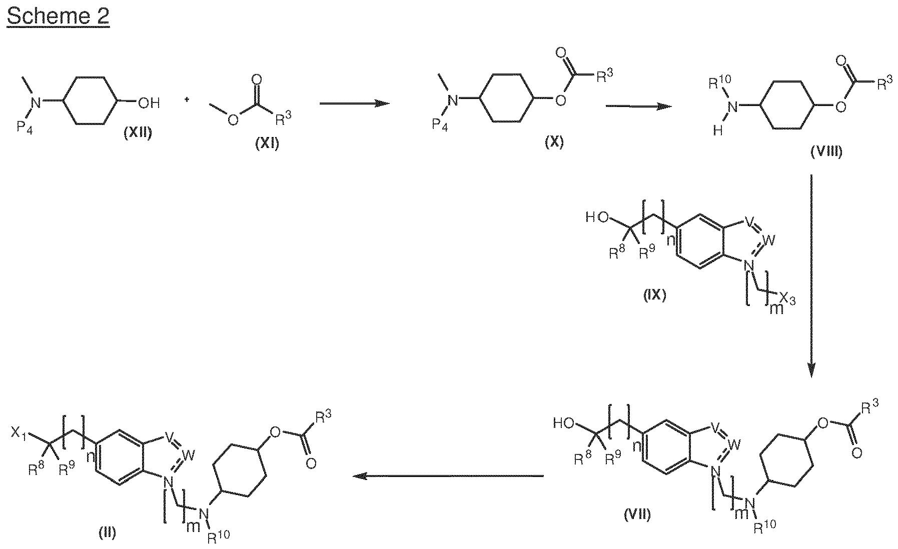

Intermediates of formula **(II)** may be prepared from alcohol derivatives of formula **(VII)** via acylation with sulphonyl halides in the presence of an acid scavenger or by halogenation with a variety of halogenating agents.

Intermediates of formula **(VII)** may be prepared by direct alkylation of an amine of formula **(VIII)** with the corresponding alkylating fragment **(IX)** wherein X₃ represents a leaving group such as a halogen atom or an active ester as mesylate or tosylate, in the presence of an acid scavenger such as a tertiary amine.

The amino-ester derivatives of formula **(VIII)** may be prepared by deprotecting compounds of formula **(X),** wherein P₄ represents a protecting group, for example, by removing tert-butoxycarbonyl group (BOC) in the presence of acidic media such as hydrogen chloride in THF.

Intermediates of formula **(X)** may be prepared by a transesterification process starting from literature-known aminoalcohol derivatives of formula **(XII)** and methyl esters derivative of formula **(XI),** typically in the presence of a base as sodium hydride and and by displacing the equilibrium by distillation of a solvent like toluene.

Intermediates of formula **(III)** are widely described in the literature (see, for example, US2004242622 example 6; WO2008149110 intermediate 65; US2007249674 example 3B), and may be prepared following the same synthetic procedure described therein.

Intermediates of formula **(IV)** may be prepared from commercially available starting materials and reagents using well known procedures, as depicted in Scheme 3. 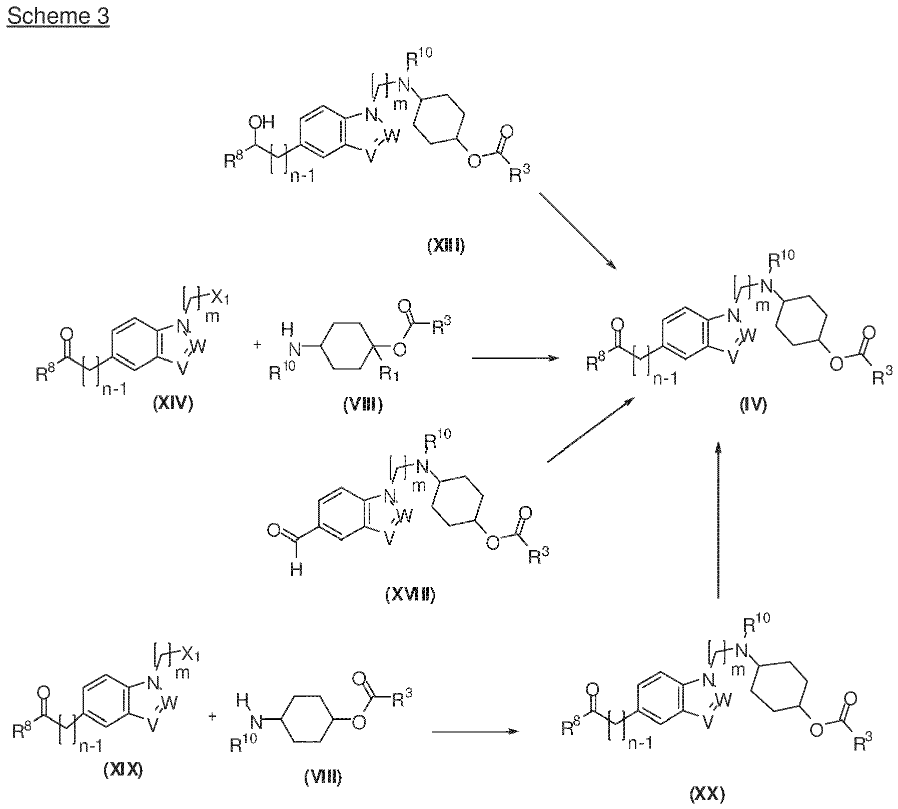 Intermediates of formula **(IV)** may be prepared either by oxidation of intermediates of formula **(XIII)** with an oxidizing agent such as manganese dioxide or Dess-Martin reagent or by direct alkylation of an intermediate of formula **(VIII)** with an alkylating agent of formula **(XIV)** in the presence of an acid scavenger.

Compounds **(IV)** wherein n=2 are also available by homolagation of aldehydes **(XVIII)** through reaction with methoxymethyltriphenylphosphine in the presence of a base such as lithium bis(trimethylsilyl)amidure and subsequent acidic hydrolysis of the intermediate enolic ether or by oxidation of the vinyl derivatives **(XX),** prepared in turn by alkylation of **(VIII)** with intermediates **(XIX).** This oxidation can be accomplished with a variety of agents, such as osmium tetroxide in the presence of N-methylmorpholine N-oxide.

Intermediates of formula **(V)** may be prepared from their N-protected homologues **(XV)** by a specific deprotecting process such as the treatment of N-BOC derivative with acidic media like hydrogen chloride in THF, as depicted in Scheme 4.

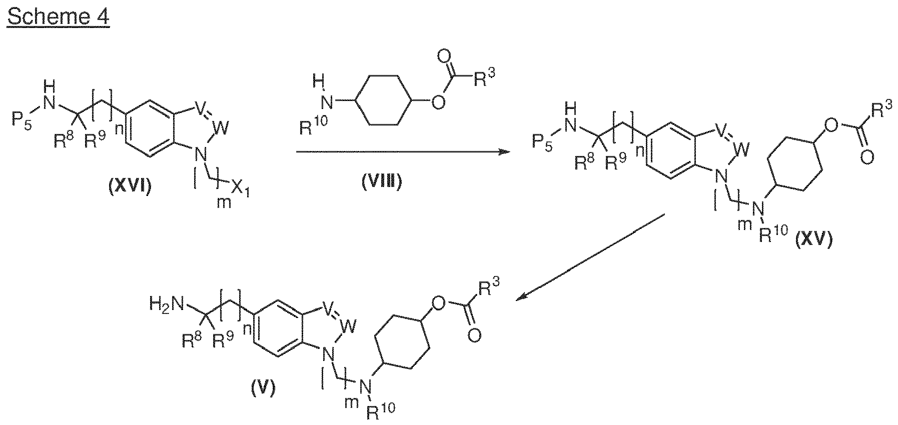

Intermediates of formula **(XV)** are in turn prepared from intermediates of formula **(VIII)** by procedures well known in the art, such as alkylation procedures with intermediates of formula **(XVI)** in the presence of an acid scavenger such as a tertiary amine. Intermediates **(XVIII)** are obtained from known compounds as depicted in Scheme 5. 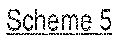 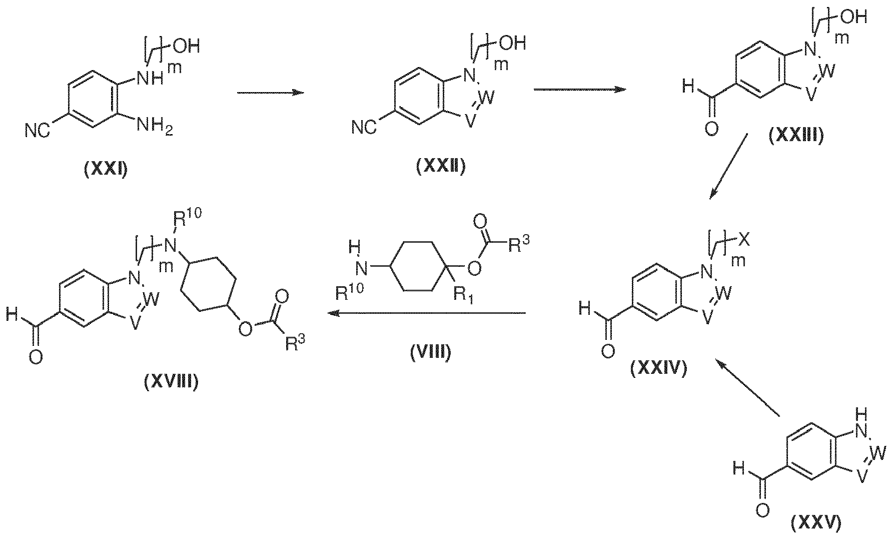 Compounds of formula **(XXI)** are transformed into the corresponding benzoimidazolones **(XXII)** (wherein W represents a -CO- and V represents a -NH-) by treatment with carbonylimidazole or triphosgene, or alternatively are transformed into the corresponding benzotriazoles **(XXII)** (wherein both W and V are -N-) by treatment with sodium nitrite in an acidic medium. Reduction of intermediate nitriles **(XXII)** with NiAl alloy in formic acid give raise to the intermediate aldehydes **(XXIII),** which in turn are transformed into the alkylating agents **(XXIV)** (X represents halide or active ester) and finally reacted with intermediate (VIII) to give intermediates **(XVIII).** Intermediates **(XXIV)** wherein W represents -CO- and V represent an oxygen atom, can also be obtained by direct N-alkylation of intermediates **(XXV)** with an α,ω-dihaloalkane in the presence of an acid scavenger.

### EXAMPLES

**General.** Reagents, starting materials, and solvents were purchased from commercial suppliers and used as received. Concentration refers to evaporation under vacuum using a Büchi rotatory evaporator. Reaction products were purified, when necessary, by flash chromatography on silica gel (40-63 µm) with the solvent system indicated or using preparative HPLC conditions (see bellow description of two systems used). Spectroscopic data were recorded on a Varian Gemini 300 spectrometer. HPLC-MS were performed on a Gilson instrument equipped with a Gilson piston pump 321, a Gilson 864 vacuum degasser, a Gilson liquid handler 215, a Gilson 189 injection module, a Gilson Valvemate 7000, a 1/1000 splitter, a Gilson 307 make-up pump, a Gilson 170 diode array detector, and a Thermoquest Finnigan aQa detector.

### HPLC systems 1:

C-18 reversed phase column silica from MERCK, water/acetonitrile as eluents [0.1% v/v ammonium formate buffered] using a gradient from 0% to 100%.

### HPLC system 2:

C-18 reversed phase column silica from MERCK, water/acetonitrile (without buffer) as eluents using a gradient from 0% to 100%.

### Intermediate 1.

### tert-butyl (trans-4.hydroxycyclohexyl)carbamate

To a solution of (1*R*,4*R*)-4-aminocyclohexanol (15 g, 0.13 mol) in acetonitrile (240 mL) was added in portions di-*tert*-butyl dicarbonate (31.2 g, 0.14 mol). The mixture was stirred overnight at room temperature. The precipitate obtained was washed with hexane/ethyl acetate (3:1) and hexane giving the title compound as a white solid (83%).
¹H NMR (300 MHz, CHLOROFORM-*d*) δ ppm 1.17 (br. s., 2 H) 1.44 (br. s., 9 H) 1.32 - 1.40 (m, 2 H) 1.99 (br. s., 4 H) 3.44 (br. s., 1 H) 3.61 (br. s., 1 H) 4.38 (br. s., 1 H)

### Intermediate 2.

### trans-4-(Methylamino)cyclohexanol

To a mixture of lithium aluminium hydride (9 g, 0.23 mol) in tetrahydrofuran (425 mL) was added slowly *tert*-butyl (*trans*-4-hydroxycyclohexyl)carbamate (intermediate 1, 10 g, 0.046 mol). The mixture was refluxed overnight. Once the mixture was cooled to room temperature, 9 ml of water, 9 ml of 4N NaOH solution and 18 ml of water were carefully and successively dropped. The organic solvent was removed under reduced pressure and the crude obtained was dissolved with chloroform and dried over magnesium sulphate. The filtrate was evaporated to dryness and co evaporated with hexane to give the title compound as a white solid (89%). This intermediate is also described in JMC, 1987, 30(2), p313*.*
¹H NMR (300 MHz, CHLOROFORM-*d*) δ ppm 1.04 - 1.20 (m, 2 H) 1.25 - 1.40 (m, 2 H) 1.97 (br. s., 4 H) 2.27 - 2.40 (m, 1 H) 3.57 - 3.70 (m, 1 H)

### Intermediate 3.

### tert-butyl (trans-4.hydroxycyclohexyl)methylcarbamate

To a solution of *trans*-4-(methylamino)cyclohexanol (intermediate 2, 5.3 g, 0.04 mol) in acetonitrile (92 mL) was added in portions di-*tert*-butyl dicarbonate (9.9 g, 0.04 mol). The mixture was stirred overnight at room temperature. The solvent was removed under reduced pressure and the crude was purified by column chromatography with silica gel, eluting with a mixture of chloroform/methanol (from 75:1 to 4:1)) to give the title compound as a colourless oil (87%).
¹H NMR (300 MHz, CHLOROFORM-*d*) δ ppm 1.34 - 1.43 (m, 2 H) 1.46 (s, 9 H) 1.49 - 1.57 (m, 2 H) 1.70 (d, *J*=9.89 Hz, 2 H) 2.03 (br. s., 3 H) 2.71 (br. s., 3 H) 3.57 (br. s., 1 H)

### Intermediate 4.

### trans-4-[(tert-butoxycarbonyl)(methyl)amino]cyclohexyl hydroxy(di-2-thienyl)-acetate

To a solution of methyl hydroxy(di-2-thienyl)acetate (5.8 g, 0.02 mol) (prepared according to Acta Chemica Scandinavica 24 (1970) 1590-1596) in anhydrous toluene (95 mL) was first added a solution of *tert*-butyl (*trans*-4-hydroxycyclohexyl)-methylcarbamate (intermediate 3; 6 g, 0.02 mol) in anhydrous toluene (95 mL) and secondly sodium hydride (60%, 0.45 g, 0.01 mol). After few minutes the mixture was warmed to 155°C and the solvent was distilled and simultaneously replaced. This procedure was carried on during 1 hour and a half. The mixture was cooled to room temperature and diluted with ether (300 mL). The organic layer was washed with sodium bicarbonate 4% (2 x 200 mL) and brine, dried, filtered and evaporated over reduced pressure giving the title compound as a yellow solid (69%), which was used in the next step without further purification.

LRMS (m/z): 452 (M+1)⁺.

### Intermediate 5.

### trans-4-(methylamino)cyclohexyl hydroxy(di-2-thienyl)acetate

To a solution of *trans*-4-[(*tert*-butoxycarbonyl)(methyl)amino]cyolohexyl hydroxy(di-2-thienyl)acetate (intermediate 4; 8.1 g, 0.01 mol) in dioxane (13.5 mL) was added hydrogen chloride 4 in dioxane (27mL). The mixture was stirred at room temperature for 24 hours. The precipitate obtained was filtrated and washed with ether. The crude was dissolved in water and potassium carbonate was added until pH=8-9. The product was extracted with ethyl and the organic layer was washed with brine, dried and evaporated to dryness giving the title compound as a white solid (78%).

LRMS (m/z): 352 (M+1)⁺.
¹H NMR (300 MHz, CHLOROFORM-*d*) δ ppm 1.14 - 1.30 (m, 2 H) 1.42 - 1.57 (m, 2 H) 1.88 - 2.11 (m, 4 H) 2.36 - 2.48 (m, 1 H) 3.71 (s, 3 H) 4.82 - 4.95 (m, 1 H) 6.94 - 7.00 (m, 2 H) 7.14 - 7.19 (m, 2 H) 7.25 - 7.30 (m, 2 H)

### Intermediate 6.

### 2-oxo-2,3-dihydro-1,3-benzoxazole-6-carbonitrile

A mixture of 6-bromo-1,3-benzoxazol-2(3H)-one (2 g; 9.34 mmol) and copper (I) cyanide (1.42 g; 15.86 mmol) in 6 ml DMF is heated at 150°C under nitrogen atmosphere for 22 hr. After cooling to room temperature, a solution of 1.55 g (31.6 mmol) of sodium cyanide in 32 ml water is added followed by 1 hr stirring. The system is extracted thoroughly with ethyl acetate, washed with brine, dried and concentrated in vacuo to provide 1.5 g (93 % yield) of the title compound enough pure as to prosecute the syntesis.

### Intermediate 7

### 2-oxo-2,3-dihydro-1,3-benzoxazole-6-carbaldehyde

A mixture of 2-oxo-2,3-dihydro-1,3-benzoxazole-6-carbonitrile (Intermediate 6, 220 mg; 1.37 mmol) and aluminium/nickel 1:1 alloy (223.6 mg; 2.61 mmol) in 2.25 ml of formic acid and 0.75 ml of water is stirred at 90ºC for 24 hr. The solid is filtered and washed with ethanol. The filtrate is concentrated in vacuo and dried overnight at 45ºC in a vacuum dessicator. The solid obtained (219 mg; 97 % yield) is pure enough as to prosecute with the synthesis.

### Intermediate 8.

### 3-(3-bromopropyl)-2-oxo-2,3-dihydro-1,3-benzoxazo!e-6-carbaldehyde

A mixture of 290 mg (1.64 mmol) of 2-oxo-2,3-dihydro-1,3-benzoxazole-6-carbaldehyde (Intermediate 7), 272.8 mg (1.96 mmol) of 3-bromo-propan-1-ol, 514 mg (1.96 mmol) of triphenylphospine and 0.855 ml (1.96 mmol) of 40 % toluene solution of DEAD in 7 ml THF is stirred overnight. After concentration in vacuo the residue is chromatographically purified over silicagel eluting with hexane/ethyl ether (100/0 to 0/100), obtaining 423 mg of the title product (58 % purity; 52 % total yield) that is used per se in the next synthetic step.

### Intermediate 9

### trans-4-[[3-(6-formyl-2-oxo-1,3-benzoxazol-3(2H)-yl)propyl](methyl)amino] cyclohexylhydroxy(di-2-thienyl)acetate

A solution of 418 mg (0.85 mmol) of 58 % pure 3-(3-bromopropyl)-2-oxo-2,3-dihydro-1,3-benzoxazole-6-carbaldehyde (Intermediate 8), 250 mg (0.71 mmol) of trans-4-(methylamino)cyclohexyl hydroxy(di-2-thienyl)acetate (Intermediate 5) and 0.14 ml (1.01 mmol) of triethylamine in 8 ml acetonitrile and 6 ml THF is heated to 90ºC under argon atmosphere for 44 hr. After concentration in vacuo the residue is chromatographically purified over silicagel eluting with dichloromethane/EtOH (from 100/0 to 80/20), obtaining 376 mg of the title product (51 % purity; 48 % total yield) that is used per se in the next synthetic step.

### Intermediate 10.

### trans-4-[{3-[6-({[(2R)-2-{[tert-butyl(dimethyl)silyl]oxy}-2-(8-hydroxy-2-oxo-1,2-dihydroquinolin-5-yl)ethyl]amino}methyl)-2-oxo-1,3-benzoxazol-3(2H)-yl]propyl} (methyl)amino]cyclohexyl hydroxy(di-2-thienyl)acetate

A mixture of 370 mg (0.34 mmol) of 51 % pure trans-4-[[3-(6-formyl-2-oxo-1,3-benzoxazol-3(2H)-yl)propyl](methyl)amino]cyclohexylhydroxy(di-2-thienyl)acetate (Intermediate 9), 167 mg (0.42 mmol) of 5-((1R)-2-amino-1-{[tert-butyl(dimethyl)-silyl]oxy}ethyl)-8-hydroxyquinolin-2(1H)-one acetate (prepared according to preparation 8 from US20060035931), 0.075 ml (0.43 mmol) of diisopropilethylamine and 379 mg (1.79 mmol) of sodium triacetoxyborohydride in 2 ml MeOH and 1 ml THF is stirred under argon atmosphere for 24 hr at room temperature. After adding 20 ml of 4% aqueous solution of sodium hydrogen carbonate the system is extracted thrice with ethyl and the organic solution washed thoroughly with 4% aqueous sodium hydrogen carbonate. After dryiyng and concentrating, the residue is chromatographically purified over silicagel eluting with chloroform/EtOH (100/0 to 0/100). 133 mg of 88 % pure title compound are obtained (39 % yield).

### EXAMPLE 1.

### trans-4-[{3-[6-({[(2R)-2-hydroxy-2-(8-hydroxy-2-oxo-1,2-dihydroquinolin-5-yl)ethyl] amino}methyl)-2-oxo-1,3-benzoxazol-3(2H)-yl]propyl}(methyl)amino]cyclohexyl hydroxy(di-2-thienyl)acetate, dihydrofluoride.

115 mg (0.12 mmol) of 88% pure trans-4-[{3-[6-({[(2R)-2-{[tert-butyl(dimethyl)silyl]oxyl-2-(8-hydroxy-2-oxo-1,2-dihydroquinolin-5-yl)ethyl]amino}methyl)-2-oxo-1,3-benzoxazol-3(2H)-yl]propyl} (methyl)amino]cyclohexyl hydroxy(di-2-thienyl)acetate (Intermediate 10) are dissolved in 3 ml THF. 0.075 ml (0.46 mmol) of triethylamine trihydrofluoride are added and the system stirred overnight at room temperature. The supernatant is discarded and the residue is washed (ultrasound bath) with 5 additional ml of THF and the supernatant is again discarded. Acetonitrile (5 ml) is added to the residue and after some stirring the solid is filtered and washed with acetonitrile and ethyl ether. 87 mg (94 % yield) of pure title compound are obtained.

LRMS (m/z): 759 (M+1)⁺.
¹H NMR (400MHz, DMSO-*d*₆) δ ppm:1.34 (m.4H); 1.61-1.72 (b.s.2H); 1.80 (t.2H); 1.85-1.96 (b.s. 2H); 2.11 (s. 3H); 2.32-2.51 (b.s. 3H); 2.66-2.76 (b.s. 2H); 3.77-3.90 (c.s. 5H); 4.62-4.74 (b.s. 1H); 5.09-5.17 (b.s. 1 H); 6.47 (d. J=12 Hz 1 H); 6.89-7.01 (c.s. 3H); 7.04-7.09 (c.s. 3H); 7.19-7.29 (c.s. 4H); 7.36 (s. 1 H); 7.44-7.49 (m. 2H); 8.13 (d. J=12 Hz 2H); 10.21-10.54 (b.s. 1 H).

### Intermediate 11.

### 4-[(3-hydroxypropyl)amino]-3-nitrobenzonitrile

A mixture of 4-fluoro-3-nitrobenzonitrile (1.0 g; 6.02 mmol), 0.502 ml (6.62 mmol) of 3-amino-propan-1-ol and 1.15 ml (6.62 mmol) of diisopropylethylamine in 5 ml THF is stirred at rt for 1 hr (temperature raises somewhat at the beginning). After concentration in vacuo the residue is dissolved in 50 ml of ethyl acetate, washed with 50 ml of 4% aqueous sodium hydrogen carbonate and brine, dried and concentrated. 1.32 g of pure title compound as a solid are thus obtained (99 % yield).

### Intermediate 12.

### 3-amino-4-[(3-hydroxypropyl)amino]benzonitrile

A mixture of 1.12 g (5.06 mmol) of 4-[(3-hydroxypropyl)amino]-3-nitrobenzonitrile (Intermediate 11) and 26.94 mg of 10% Pd on charcoal in 39 ml of EtOH is shaken in an hydrogen atmosphere (14 psi) for 20 hr at rt. After filtration and evaporation 1.012 g of the pure title compound are obtained.

### Intermediate 13.

### 1-(3-hydroxypropyl)-2-oxo-2,3-dihydro-1H-benzimidazole-5-carbonitrile

100 mg (0.52 mmol) of 3-amino-4-[(3-hydroxypropyl)amino]benzonitrile (Intermediate 12) are dissolved in 2.5 ml of 2N aqueous HCl and 1.5 ml of toluene are added. 150 mg (0.51 mmol) of triphosgene are added and the system is stirred at rt for 18 hr. After adding 75 additional mg of triphosgene and prosecuting the stirring for 1 hour 5 ml of brine and 25 ml of ethyl acetate are added and the system is stirred for 10 minutes. The organic layer is isolated and the aqueous one is extracted with 4x10 ml of ethyl The combined organic phases are washed with brine, dried and concentrated to give 106 mg (93 % yield) of 100 pure (UPLC) title compound.

### Intermediate 14.

### 1-(3-hydroxypropyl)-2-oxo-2,3-dihydro-1H-benzimidazole-5-carbaldehyde

A mixture of 930 mg (4.28 mmol) of 1-(3-hydroxypropyl)-2-oxo-2,3-dihydro-1H-benzimidazole-5-carbonitrile (Intermediate 13) and 949 mg of Ni-Al alloy 1:1 in 7.3 ml of 75 % formic acid in water is stirred at 90ºC for 6.5 hr. After filtration, the residue is again dissolved in 7.3 ml of 75 % formic acid, 949 mg of Ni-Al alloy are added and the system is stirred at 90º C for 1 hr. After filtration, 5 ml of 2N NaOH and 5 ml of EtOH are added and the system is stirred at rt overnight. The pH is made 6-7 by addition of 2N HCl and the solution is concentrated. The residue is chromatographically purified over silicagel eluting with hexane/EtOH (100/0 to 0/100). 0.91 g of pure title compound are obtained (96 % yield).

### Intermediate 15.

### 1-(3-bromopropyl)-2-oxo-2,3-dihydro-1H-benzimidazole-5-carbaldehyde

1185 mg (5.38 mmol) of 1-(3-hydroxypropyl)-2-oxo-2,3-dihydro-1H-benzimidazole-5-carbaldehyde (Intermediate 14) are suspended in 56 ml of dichloromethane. 2285 mg (6.89 mmol) of carbon tetrabromide and then 6890 mg of polymer-supported triphenyl phosphine (1 mmol/g; 6.89 mmol) are added. The mixture is shaken at rt for 24 hr. The polymer is filtered and sequentially washed with dichloromethane, EtOH and MeOH. The filtrates are concentrated and the residue (2.2 g) is chromatographically purified over silicagel eluting with chloroform/EtOH (100/0 to 90/10). 0.3 g of pure title compound are obtained (20 % yield).

### Intermediate 16.

### trans-4-[[3-(5-formyl-2-oxo-2,3-dihydro-1H-benzimidazol-1-yl)propyl](methyl) amino] cyclohexyl hydroxy(di-2-thienyl)acetate

A mixture of 179 mg (0.56 mmol) of 1-(3-bromopropyl)-2-oxo-2,3-dihydro-1H-benzimidazole-5-carbaldehyde (Intermediate 15), 163 mg (0.46 mmol) of trans-4-(methylamino)cyclohexyl hydroxy(di-2-thienyl)acetate (Intermediate 5) and 0.09 ml (0.65 mmol) of triethylamine in 6 ml acetonitrile and 4 ml THF is stirred at 90ºC overnight. After concentration the residue (325 mg) is chromatographically purified over silicagel eluting with hexane to ethyl ether/EtOH 90/10 and again with C-18 reversed phase column silica from MERCK, using water to acetonitrile/MeOH as eluents with a gradient from 0% to 100%. 94 mg of pure title compound are thus obtained (36 % yield).

### Intermediate 17.

### trans-4-[{3-[5-({[(2R)-2-{[tert-butyl(dimethyl)silyl]oxy}-2-(8-hydroxy-2-oxo-1,2-dihydroquinolin-5-yl)ethyl]amino}methyl)-2-oxo-2,3-dihydro-1H-benzimidazol-1-yl]propyl}(methyl)amino]cyclohexyl hydroxy(di-2-thienyl)acetate

85 mg (0.15 mmol) of trans-4-[[3-(5-formyl-2-oxo-2,3-dihydro-1H-benzimidazol-1-yl)propyl](methyl) amino] cyclohexyl hydroxy(di-2-thienyl)acetate (Intermediate 16) and 80 mg (0.20 mmol) of 5-((1R)-2-amino-1-{[tert-butyl(dimethyl)-silyl]oxy}ethyl)-8-hydroxyquinolin-2(1H)-one acetate (prepared according to preparation 8 from US20060035931) are dissolved in 2 ml MeOH and 1 ml THF. After adding 0.04 ml (0.23 mmol) of diisopropylethylamine and 100 mg (0.47 mmol) of sodium triacetoxyborohydride the mixture is stirred overnight under argon atmosphere at rt. After adding 100 mg more of sodium triacetoxyborohydride the stirring is prosecuted for 48 hr. After concentrating in vacuo the residue is partitioned in ethyl acetate/4% aqueous sodium hydrogen carbonate solution. A yellowish solid is filtered, dissolved in chloroform and washed with 4 % NaHCO3. The combined organic phases are dried and concentrated. The residue is chromatographically purified over silicagel eluting with chloroform/EtOH/Et3N (100/0/0.1 to 0/100/0.1). 96 mg of 71 % pure title compound are obtained (51 % yield) and used per se in the next synthetic step.

### EXAMPLE 2.

### trans-4-[{3-[5-({[(2R)-2-hydroxy-2-(8-hydroxy-2-oxo-1,2-dihydroquinolin-5-yl)ethyl]amino}methyl)-2-oxo-2,3-dihydro-1H-benzimidazol-1-yl]propyl} (methyl)amino]cyclohexyl hydroxy(di-2-thienyl)acetate dihydrofluoride.

95 mg (0.12 mmol) of 71% pure trans-4-[{3-[5-({[(2R)-2-{[tert-butyl(dimethyl)silyl]oxy}-2-(8-hydroxy-2-oxo-1,2-dihydroquinolin-5-yl)ethyl]amino}methyl)-2-oxo-2,3-dihydro-1H-benzimidazol-1-yl]propyl}(methyl)amino]cyclohexyl hydroxy(di-2-thienyl)acetate (Intermediate 17) are dissolved in 2 ml THF. 0.05 ml (0.31 mmol) of triethylamine trihydrofluoride are added and the system stirred overnight at room temperature. The supernatant is discarded and the residue is washed with 2x10 additional ml of THF and the supernatant is again discarded. Acetonitrile (5 ml) is added to the residue and the solid is stirred for 2 hr, aged overnight, filtered and washed with acetonitrile. The residue is chromatographically purified with C-18 reversed phase column silica from MERCK, using water to acetonitrile/MeOH as eluents with a gradient from 0% to 100%. 31.8 mg of pure title compound are thus obtained (51 % yield).

LRMS (m/z):758 (M+1)⁺.
¹H NMR (400 MHz, DMSO-*d*₆) δ ppm: 1.35 (m. 6H); 1.61-1.77 (b.s. 5H); 1.85-1.97 (b.s. 2H); 2.12 (s. 3H); 2.32-2.45 (b.s. 4H); 2.68-2.74 (b.s. 2H); 3.72-3.86 (c.s. 3H); 4.62-4.73 (b.s. 1H); 5.06-5.14 (b.s. 1H); 6.47 (d. J=12 Hz 1H); 6.90 (d. J=6Hz 1 H); 6.95-7.09 (c.s. 8H); 7.23-7.30 (b.s. 1H); 7.47 (d. J=6Hz 1 H); 8.06 (d. J=12 Hz 1H); 10.26-10.49 (b.s. 1H); 10.80-10.88 (b.s. 1H).

### Intermediate 18.

### 1-(3-hydroxypropyl)-1H-1,2,3-benzotriazole-5-carbonitrile

100 mg (0.52 mmol) of 3-amino-4-[(3-hydroxypropyl)amino]benzonitrile (Intermediate 12) are suspended in 0.5 ml of 5N aqueous HCl. After cooling externally with an ice/water bath, a solution of 54.12 mg (0.78 mmol) of sodium nitrite in 0.4 ml water is added dropwise with stirring. After 3.5 hr excess water is added and the solid is extracted with dichloromethane, washed with water, dried and concentrated to give 104 mg of pure title compound (96 % yield).

### Intermediate 19.

### 1-(3-hydroxypropyl)-1H-1,2,3-benzotriazole-5-carbaldehyde

A mixture of 500 mg (2.47 mmol) of 1-(3-hydroxypropyl)-1H-1,2,3-benzotriazole-5-carbonitrile (Intermediate 18) and 550 mg of Ni-Al alloy 1:1 in 5.55 ml of 75 % formic acid in water is stirred at 90ºC for 2.5 hr. After filtration and evaporation 10 ml of 2N NaOH and 10 ml of EtOH are added to the residue and the system is stirred at rt for 1.5 hr. The pH is made 6-7 by addition of 2N HCl and the system is extracted thoroughly with ethyl acetate. After washing with water, drying and concentrating, 0.35 g of 80 % pure title compound (55 % yield).

### Intermediate 20.

### 1-(3-bromopropyl)-1H-1,2,3-benzotriazole-5-carbaldehyde

200 mg (0.975 mmol) of 1-(3-bromopropyl)-1H-1,2,3-benzotriazole-5-carbaldehyde (Intermediate 19) are dissolved in 10.5 ml of dichloromethane. 388 mg (1.17 mmol) of carbon tetrabromide are added and the solution cooled externally with an ice/water bath. 307 mg (1.17 mmol) of triphenylphosphine are slowly added and the system is stirred for 20 min with external cooling and 2 hr at rt. After addition of 0.5 more equivalents of both carbon tetrabromide and triphenylphosphine and additional stirring for 10 min with the external cooling and 1 hr at rt the solvents are eliminated in vacuo and the residue is chromatographically purified over silicagel eluting with hexane/ethyl ether (100/0 to 0/100). 195 mg of pure title compound are obtained (74 % yield).

### Intermediate 21.

### trans-4-[[3-(5-formyl-1H-1,2,3-benzotriazol-1-yl)propyl](methyl)amino]cyclohexyl hydroxy(di-2-thienyl)acetate

A mixture of 152.56 mg (0.57 mmol) of 1-(3-bromopropyl)-1H-1,2,3-benzotriazole-5-carbaldehyde (Intermediate 20), 200 mg (0.57 mmol) of trans-4-(methylamino) cyclohexyl hydroxy(di-2-thienyl)acetate (Intermediate 5) and 0.138 ml 0.80 mmol) of diisopropylethylamine in 25 ml acetonitrile was stirred under argon at 75ºC for 17 hr and at 90ºC for 24 hr. After concentration in vacuo, the residue is chromatographically purified over silicagel eluting with chloroform/EtOH (100/0 to 90/10) to give 157 mg of pure title compound (51 % yield).

### Intermediate 22.

### trans-4-[{3-[5-({[(2R)-2-{[tert-butyl(dimethyl)silyl]oxy}-2-(8-hydroxy-2-oxo-1,2-dihydroquinolin-5-yl)ethyl]amino}methyl)-1H-1,2,3-benzotriazol-1-yl]propyl} (methyl)amino]cyclohexyl hydroxy(di-2-thienyl)acetate

132 mg (0.245 mmol) of trans-4-[[3-(5-formyl-1H-1,2,3-benzotriazol-1-yl)propyl](methyl)amino]cyclohexyl hydroxy(di-2-thienyl)acetate (Intermediate 21) and 106.3 mg (0.269 mmol) of 5-((1R)-2-amino-1-{[tert-butyl(dimethyl)-silyl]oxy}ethyl)-8-hydroxyquinolin-2(1H)-one acetate (prepared according to preparation 8 from US20060035931) are dissolved in 1.6 ml MeOH and 0.8 ml THF. After adding 0.05 ml (0.29 mmol) of diisopropylethylamine and 76.9 mg (0.36 mmol) of sodium triacetoxyborohydride the mixture is stirred overnight under argon atmosphere at rt. After successive addition of 230 additional mg (1.08 mmol) of reducing agent, stirring for 3 hr and 76.9 mg (0.36 mmol) more and stirring for 2 hr the solvents are eliminated in vacuo and the residue (0.57 g) is stirred with chloroform, filtered and the solid discarded. The filtrate is concentrated and partitioned between 50 ml of ethyl acetate and 10 ml of 4% solution of sodium hydrogen carbonate. The organic solution is washed again with NaHCO3 solution, dried and concentrated to give 210 mg of 91 % pure title compound (yield 91%) that is used per se in the next synthetic step.

### EXAMPLE 3.

### trans-4-[{3-[5-({[(2R)-2-hydroxy-2-(8-hydroxy-2-oxo-1,2-dihydroquinolin-5-yl)ethyl]amino}methyl)-1H-1,2,3-benzotriazol-1-yl]propyl}(methyl)amino] cyclohexyl hydroxy(di-2-thienyl)acetate dihydrofluoride.

205 mg (0.218 mmol) of 91% pure trans-4-[{3-[5-({[(2R)-2-{[tert-butyl(dimethyl) silyl]oxy}-2-(8-hydroxy-2-oxo-1,2-dihydroquinolin-5-yl)ethyl]amino}methyl)-1H-1,2,3-benzotriazol-1-yl]propyl}(methyl)amino]cyclohexyl hydroxy(di-2-thienyl) acetate (Intermediate 22) are dissolved in 2 ml THF. 0.145 ml (0.89 mmol) of triethylamine trihydrofluoride are added and the system stirred overnight at room temperature. The supernatant is discarded and the residue is washed with 2x3 additional ml of THF and the supernatant is again discarded. Acetonitrile (4 ml) is added to the residue and the solid is stirred for 30 min, filtered and washed with more acetonitrile. After drying overnight at 40ºC 164 mg of pure title compound are thus obtained (96 % yield).

LRMS (m/z): 743 (M+1)⁺.
¹H NMR (400 MHz, DMSO-*d*₆) δ ppm: 1.32 (m. 5H); 1.59-1.69 (b.s. 3H); 1.84-1.94 (b.s. 2H); 2.01 (m. 2H); 2.11 (s. 3H); 2.26-2.40 (c.s. 4H); 2.60-2.75 (c.s. 2H); 3.90 (s. 2H); 4.34-4.42 (b.s. 1 H); 4.67 (m. 4H); 5.04-5.11 (m. 1H); 5.28-5.46 (b.s. 1 H); 6.40 (d. J=12 Hz 1 H); 6.86-6.93 (c.s. 1 H); 6.94-7.00 (c.s. 2H); 7.03-7.10 (c.s. 4H); 7.19-7.34 (b.s. 1 H); 7.44-7.48 (m. 2H); 7.49-7.54 (m. 1 H); 7.74-7.80 (m. 1 H); 7.92 (s. 1H); 8.10 (d. J=12 Hz 1H); 10.10-10.51 (b.s. 1 H).

### Intermediate 23.

### 1-(3-bromopropyl)-1H-indole-5-carbaldehyde

0.70 g (30.31 mmol) of 60 % sodium hydride suspension are added to 14 ml of anhydrous DMF and a solution of 2.40 g (16.53 mmol) of 1H-indole-3-carbaldehyde in 10 ml of DMF added dropwise. After 45 min of stirring at room temperature the solution is cooled externally with an ice/water bath and a solution of 2.52 ml (5.01 g; 24.80 mmol) of 1,3-dibromopropane in 6 ml of DMF added dropwise. The solution is stirred at room temperature for 2 hr before adding 10 ml of water and 10 ml of 2N HCl. The suspension is extracted thrice with ethyl ether, washed with water, dried and concentrated in vacuo. The residue is chromatographically purified (hexane to hexane/EtAcO 4:1) to give 1.4 g of the pure title compound (33 % yield).

### Intermediate 24.

### trans-4-[[3-(5-formyl-1H-indol-1-yl)propyl](methyl)amino]cyclohexyl hydroxy(di-2-thienyl)acetate

A mixture of 1.27 g (4.78 mmol) of 1-(3-bromopropyl)-1H-indole-5-carbaldehyde (intermediate 23), 1.40 g (3.98 mmol) of trans-4-(methylamino)cyclohexyl hydroxy(di-2-thienyl)acetate (intermediate 5) and 0.77 ml (0.56 g; 5,5 mmol) of triethylamine in 6 ml MeCN and 6 ml THF is stirred at 90ºC overnight under argon. After concentrating in vacuo the residue is chromatographically purified eluting with C13CH to Cl3CH/MeOH 95:5 to give 1.6 g (75 % yield) of pure title compound.

### Intermediate 25.

### trans-4-[{3-[5-({[(2R)-2-{[tert-butyl(dimethyl)silyl]oxy}-2-(8-hydroxy-2-oxo-1,2-dihydroquinolin-5-yl)ethyl]amino}methyl)-1H-indol-1-yl]propyl}(methyl)amino] cyclohexyl hydroxy(di-2-thienyl)acetate

A mixture of 190 mg (0.35 mmol) of trans-4-[[3-(5-formyl-1H-indol-1-yl)propyl](methyl)amino]cyclohexyl hydroxy(di-2-thienyl)acetate (intermediate 24), 174. 59 mg (0.44 mmol) of 5-((1R)-2-amino-1-{[tert-butyl(dimethyl)-silyl]oxy}ethyl)-8-hydroxyquinolin-2(1H)-one acetate (prepared according to preparation 8 from US20060035931), 0.077 ml (0.44 mmol) of diisopropilethylamine and 243.8 mg (1.15 mmol) of sodium triacetoxyborohydride in 2 ml of MeOH and 1 ml THF is stirred under argon atmosphere for 2.5 hr at room temperature. After adding 25 ml of 4% aqueous solution of sodium hydrogen carbonate the system is extracted thrice with ethyl acetate and the organic solution washed thoroughly with 4% aqueous sodium hydrogen carbonate. After dryiyng and concentrating, the residue is chromatographically purified over silicagel eluting with chloroform/EtOH (100/0 to 90/10). 177 mg of 94 % pure title compound are obtained (55 % yield).

### EXAMPLE 4.

### trans-4-[{3-[5-({[(2R)-2-hydroxy-2-(8-hydroxy-2-oxo-1,2-dihydroquinolin-5-yl)ethyl]amino}methyl)-1H-indol-1-yl]propyl}(methyl)amino]cyclohexyl hydroxy(di-2-thienyl)acetate dihydrofluoride.

170 mg (0.20 mmol) of 91% pure trans-4-[{3-[5-({[(2R)-2-{[tert-butyl(dimethyl)silyl]oxy}-2-(8-hydroxy-2-oxo-1,2-dihydroquinolin-5-yl)ethyl]amino} methyl)-1H-1,2,3-benzotriazol-1-yl]propyl}(methyl)amino]cyclohexyl hydroxy(di-2-thienyl)acetate (Intermediate 22) are dissolved in 7 ml THF. 0.08 ml (0.78 mmol) of triethylamine trihydrofluoride are added and the system stirred overnight at room temperature. The supernatant is discarded and the residue is washed with 2x3 additional ml of THF and the supernatant is again discarded. Acetonitrile (4 ml) is added to the residue and the solid is stirred for 30 min, filtered and washed with more acetonitrile. After drying overnight at rt 146 mg of 98 % pure title compound are thus obtained (92 % yield).

LRMS (m/z): 741 (M+1)+.
¹H NMR (400 MHz, DMSO-d6) δ ppm: 1.35 (m. 5H); 1.60-1.73 (b.s. 3H); 1.79-1.95 (m. 5H); 2.00-2.16 (c.s. 4H); 2.27-2.41 (b.s. 2H); 2.66-2.83 (b.s. 3H); 3.53-3.65 (c.s. 1H); 4.00 (s. 2H); 4.15 (m. 2H); 4.66 (m. 1H); 5.17 (m. 1H); 6.40 (b.s. 2H); 6.90 (m. 1 H); 6.98 (m. 1 H); 7.07 (b.s. 3H); 7.14-7.49 (c.s. 6H); 7.55 (s. 1 H); 8.02 (d. J=12 Hz 1 H); 9.10-10.70 (b.s. 1 H).

### Biological tests

### Test 1: Human Adrenergic β₁, and β₂ Receptor Binding Assays

The study of binding to human adrenergic beta1 and beta2 receptors was performed using commercial membranes prepared from Sf9 cells where they are overexpressed (Perkin Elmer). The membrane suspensions (16 µg/well for beta1 and 5µg/well for beta2) in assay buffer (75mM Tris/HCl with 12.5mM MgCl2 and 2mM EDTA pH=7.4) were incubated with 0.14 or 0.6 nM of 3H-CGP12177 (Amersham) for beta 1 and beta 2 receptors respectively in a final volume of 250 µl, in GFC Multiscreen 96 well plates (Millipore) previously treated with assay buffer containing 0.3 % PEI (Sigma). Non specific binding was measured in the presence of 1µM propanolol. Incubation was maintained for 60 minutes at room temperature and with gentle shaking. The binding reactions were terminated by filtration and washing with 2.5 volumes of Tris/HCl 50mM pH=7.4. The affinity of each test compound to the receptor was determined by using ten different concentrations ran in duplicate. IC50s were calculated using Activity Base software from IDBS and the four parameters-log equation.

### Test 2: Human Muscarinic M₁, M₂, M₃, M₄ and M₅ receptors binding assays

The study of binding to human muscarinic M1, M2, M3, M4 and M5 receptors was performed using commercial membranes (Perkin Elmer) prepared from CHO-K1 cells. Radioligand binding experiments were conducted in 96 polypropylene well plates in a total volume of 200 µl. All reagents were dissolved in assay binding buffer (PBS with calcium and magnesium, SIGMA), except compounds that were dissolved in DMSO 100%. Non-specific binding (NSB) was measured in the presence of 1 µM atropine. [3H]-NMS was used as the radioligand at a concentration of 1 nM for M2, M3 and M5 and 0.3 nM for M1 and M4. [3H]-NMS and antagonists were incubated with membranes that express human muscarinic receptors M1, M2, M3, M4 and M5 at concentrations of 8.1, 10, 4.9, 4.5 and 4.9 µg/well, respectively.
After an incubation period of two hours with gentle shaking, 150 µl of the reaction mix were transferred to 96 GF/C filter plates (Millipore), previously treated with wash buffer (Tris 50 mM ; NaCl 100 mM; pH:7.4), containing 0.05 % PEI (Sigma) during one hour. Bound and free [3H]-NMS were separated by rapid vacuum filtration in a manifold from Millipore and washed four times with ice cold wash buffer. After drying 30 min, 30 µl of OPTIPHASE Supermix were added to each well and radioactivity quantified using a Microbeta microplate scintillation counter.

The affinity of each test compound to the receptors was determined by using ten different concentrations ran in duplicate. IC50s were calculated using Activity Base software from IDBS and the four parameters-log equation.

| **Example** | **Binding IC₅₀, nM** | |
|---|---|---|
| | **β2** | **M3** |
| 1 | 140 | 0.3 |
| 2 | 120 | 0.4 |
| 4 | 220 | 0.6 |

### PHARMACEUTICAL COMPOSITIONS

Compounds of the invention intended for pharmaceutical use may be administered as crystalline or amorphous products, or mixtures thereof. They may be obtained, for example, as solid plugs, powders, or films by methods such as precipitation, crystallization, freeze drying, spray drying, or evaporative drying.

Microwave or radio frequency drying may be used for this purpose.

Pharmaceutical compositions according to the present invention comprise the compounds of the invention in association with a Pharmaceutical acceptable diluent or carrier.

As used herein, the term pharmaceutical composition refers to a mixture of one or more of the compounds described herein, or physiologically/pharmaceutically acceptable salts, solvates, N-oxides, isomers, isotopes, polymorphs or prodrugs thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

A Pharmaceutical acceptable excipient refers to an inert substance added to a pharmaceutical composition to further facilitate administration of a compound.

The invention further provides pharmaceutical compositions comprising the compounds of the invention in association with a pharmaceutically acceptable diluent or carrier together with one or more other therapeutic agents such as the previously described for use in the treatment of a pathological condition or disease associated with both β2 adrenergic receptor agonist and muscarinic receptor antagonist activities.

The invention is also directed to pharmaceutical compositions of the invention for use in the treatment of a pathological disease or disorder associated with both β2 adrenergic receptor agonist and muscarinic receptor antagonist activities, in particular wherein the pathological disease or disorder is selected from a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis.

The invention also provides a method of treatment of a pathological condition or disease with both β2 adrenergic receptor agonist and muscarinic receptor antagonist activities in particular wherein the pathological condition or disease is selected from a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001% to 99% by weight, preferably 0.01 % to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21 st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21 st Edition, Lippincott Williams & Wilkins, Philadelphia, Pa., 2001; or in Handbook of Pharmaceutical Excipients, 6th ed., published by Pharmaceutical Press and American Pharmacists Association, 2009.

### i) Oral Administration

The compounds of the invention may be administered orally (peroral administration; **per os** (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystal line cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y., 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified Modified release formulations include delayed-, sustained-, pulsed-, controlled-, targeted and programmed release.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a Pharmaceutical acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.
Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.0001-10 mg, more preferably 0.001-2 mg of active ingredient or the equivalent amount of a pharmaceutically acceptable salt thereof. Alternatively, the active ingredient (s) may be presented without excipients.

Packaging of the formulation may be suitable for unit or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.
Pressurized aerosol compositions will generally be filled into canisters fitted with a valve, especially a metering valve. Canisters may optionally be coated with a plastics material e. g. a fluorocarbon polymer as described in WO96/32150. Canisters will be fitted into an actuator adapted for buccal delivery.

### iv) Nasal mucosal administration

The compounds of the invention may also be administered via the nasal mucosal. Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.
The amount of the active compound administered will be dependent on the subject being treated, the severity of the disorder or condition, the rate of administration, the disposition of the compound and the discretion of the prescribing physician. However, an effective dosage is typically in the range of 0.01-3000 µg, more preferably 0.5-1000 µg of active ingredient or the equivalent amount of a Pharmaceutical acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy. Preferably the composition is in unit dosage form, for example a tablet, capsule or metered aerosol dose, so that the patient may administer a single dose.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies one of the preferred application form, especially in the therapy of obstructive lung diseases or for the treatment of asthma.

### COMBINATIONS

The compounds of the present invention can also be used in combination with other drugs known to be effective in the treatment of the diseases or the disorders indicated above. For example the compounds of the present invention can be combined with a corticosteroid and/or with a PDE4 inhibitor.

Accordingly, another embodiment of the invention is a combination product comprising
(i) at least a compound as defined previously, and
(ii) one or more active ingredients selected from the group consisting of a corticosteroid and a PDE4 inhibitor,
   for simultaneous, separate or sequential use in the treatment of the human or animal body.

A preferred embodiment of the invention is a combination product as defined before for the treatment or prevention of pathological conditions, diseases and disorders associated with both β2 adrenergic receptor agonist and muscarinic receptor antagonist activities in particular wherein the pathological condition or disease is selected from from a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis; as well as a method for treating a subject afflicted with a pathological condition or disease associated with both β2 adrenergic receptor agonist and muscarinic receptor antagonist activities, in particular wherein the pathological condition or disease is selected from a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis; preferably asthma and chronic obstructive pulmonary disease, which comprises administering to said subject an effective amount of a combination product as defined before.

As indicated above, the compounds or pharmaceutically acceptable salts, solvates, N-oxides, isomers, isotopes, polymorphs or prodrugs thereof, according to the invention may also be used in combination with another therapeutically active agent, for example a corticosteroid and/or with a PDE4 inhibitor.

The amount of each active which is required to achieve a therapeutic effect will, of course, vary with the particular active, the route of administration, the subject under treatment, and the particular disease or disorder being treated.

The active ingredients may be administered from 1 to 6 times a day, sufficient to exhibit the desired activity. Preferably, the active ingredients are administered once or twice a day, most preferably once a day.

Examples of suitable corticosteroids and glucocorticoids that can be combined with the compounds of the present invention are prednisolone, methylprednisolone, dexamethasone, dexamethasone acetate, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone, mometasone furoate, rimexolone, prednisolone farnesylate, ciclesonide, butixocort propionate, RS-85095, CGP-13774, GW-250495, deltacortisone, NO-Prednisolone, NO-Budesonide, etiprednol dicloacetate, QAE-397, 7beta-OH-EPIA, RPR-106541, deprodone propionate, fluticasone, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, desisobutyrylciclesonide, hydrocortisone acetate, hydrocortisone sodium succinate, prednisolone sodium phosphate and hydrocortisone probutate, prednisolone sodium metasulfobenzoate and clobetasol propionate.

Examples of suitable PDE4 inhibitors that can be combined with the compounds of the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, revamilast, ronomilast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, K-0873, CDC-801, GSK-356278, TA-7906, CP-80633, RPL-554, NIK-616, GPD-1116, D4396, UK-500001, BLX-914, 2-carbomethoxy-4-cyano-4-(3-cyclopropylmethoxy-4-difluroromethoxyphenyl) cyclohexan1-one, cis [4-cyano-4-(3-cyclopropylmethoxy-4-difluoromethoxyphenyl) cyclohexan-1-ol, 5(S)-[3-(Cyclopentyloxy)-4-methoxyphenyl]-3(S)-(3-methylbenzyl) piperidin-2-one (IPL-455903), ONO-6126 (Eur Respir J 2003, 22(Suppl. 45): Abst 2557) and the compounds claimed in the PCT patent applications number WO 03/097613, WO 2004/058729, WO 2005/049581, WO 2005/123693, WO 2005/123692, and WO 2010/069504.

Particularly preferred combination products according to the invention comprise a compound of the present invention and a therapeutically effective amount of one or more additional therapeutic agents selected from the group consisting of mometasone furoate, ciclesonide, budesonide, fluticasone propionate, fluticasone furoate, betamethasone valerate, clobetasol propionate, rolipram, roflumilast, oglemilast, cilomilast, arofylline, apremilast and tetomilast.

Thus, in one aspect of the invention, the combination product comprises a compound of the present invention and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In a still other aspect of the invention, the combination product comprises a compound of the present invention and a PDE4 inhibitor. Particularly preferred PDE4 inhibitors are those selected from the group consisting of rolipram, roflumilast, oglemilast, cilomilast and the compounds claimed in the PCT patent applications number WO 03/097613, WO 2004/058729, WO 2005/049581, WO 2005/123693, WO 2005/123692, and WO 2010/069504. The combination product may further comprise a corticosteroid selected from the group consisting of mometasone furoate, ciclesonide, budesonide, fluticasone furoate and fluticasone propionate.

The compounds of the present invention and the combinations of the invention may be used in the treatment of respiratory, skin and inflammatory diseases, wherein the use of a dual both β2 adrenergic receptor agonist and antimuscarinic receptor antagonist is expected to have a beneficial effect, for example a pulmonary disease, such as asthma or chronic obstructive pulmonary disease, pre-term labor, glaucoma, a neurological disorder, a cardiac disorder, inflammation, urological disorders such as urinary incontinence and gastrointestinal disorders such as irritable bowel syndrome or spastic colitis; preferably asthma and chronic obstructive pulmonary disease.

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-α-day dosing occurred, or separately. Preferably at least two, and more preferably all, of the actives would be administered together at the same time. Preferably, at least two, and more preferably all actives would be administered as an admixture.

### FORMULTION EXAMPLE

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10 g |
| 1,1,1,2,3,3,3-heptafluoro-n-propane | q.s. to 200 ml |

## Claims

1. A compound of formula (A), or Pharmaceutical acceptable salts or N-oxides or solvates or deuterated derivatives thereof: 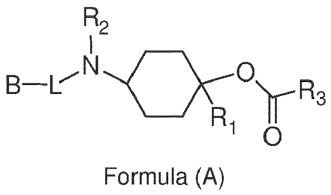 wherein
• R₁ and R₂ independently are selected from the group consisting of a hydrogen atom, a linear or branched C₁₋₄ alkyl group, a linear or branched C₁₋₄ hydroxyalkyl group and a linear or branched C₁₋₄ alkoxy group,
• R₃ represents a group of formula: 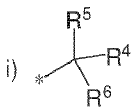 or 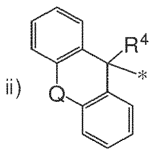 wherein:
○ R⁴ represents a hydrogen atom, a hydroxy group, a C₁₋₄ hydroxyalkyl group, a linear or branched C₁₋₄ alkyl group or a linear or branched C₁₋₄ alkoxy group,
○ R⁵ represents a saturated or unsaturated C₃₋₈ cycloalkyl group, C₅₋₆ aryl group, a 5- to 6- membered heteroaryl group containing at least one heteroatom selected from N, S, and O; a (C₁₋₄ alkyl)-(C₅₋₆ aryl) group, a (C₁₋₄ alkyl)-(C₃₋₈ cycloalkyl) group or a (C₁₋₄ alkyl)-(5 to 6 membered heteroaryl group containing at least one heteroatom selected from N, S, and O) group, which groups independently are optionally substituted with one or more substituents R^{a},
○ R⁶ represents a C₅₋₆ aryl group, a 5- to 6- membered heteroaryl group containing at least one heteroatom selected from N, S, and O, a saturated or unsaturated C₃₋₈ cycloalkyl group, a linear or branched C₁₋₈ alkyl group, a C₂₋₈ alkenyl group, a C₂₋₈ alkynyl group, a linear or branched C₁₋₈ alkoxy group, a (C₁₋₄ alkyl)-(C₅₋₆ aryl) group, a (C₁₋₄ alkyl)-(C₃₋₈ cycloalkyl) group or a (C₁₋₄ alkyl)-(5- to 6- membered heteroaryl group containing at least one heteroatom selected from N, S, and O) group which groups independently are optionally substituted with one or more substituents R^{b},
○ R^{a} and R^{b} independently represent a halogen atom, a hydroxy group, a C₁₋₄ alkyl group, C₁₋₄ alkoxy group, -SH, a C₁₋₄ alkylthio group, a nitro group, a cyano group, -CO₂R', -NR'R"', -C(O)NR'R", -N(R"')C(O)-R', -N(R'")-C(O)NR'R", whererin R', R" and R'" each independently represents a hydrogen atom or a C₁₋₄ alkyl group, or R' and R" together with the nitrogen atom to which they are attached from a 3 to 6 membered heterocyclic ring,
○ Q represents a direct bond, a -CH₂-, -CH₂-CH₂-, -O-, -O-CH₂-, -S-, -S-CH₂-, -NH-, -NH-CH₂- or -CH=CH-,
○ * represents the point of attachment of R₃ to the remainder of the molecule of formula (A),
○ L is a suitable covalent linker, and
○ B is a moiety having a beta2-adrenergic binding activity,

2. A compound according to claim 1, wherein the linker L has the following formula (L): 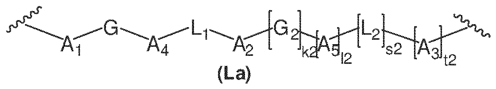 or 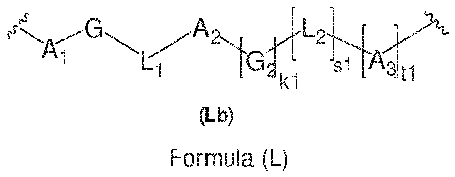 wherein k1, k2, s1, s2, l2, t1 and t2 independently have a value of 0 and 1;
• A₁, A₂, A₃, A₄ and A₅ each independently are selected from the group consisting of a C₁₋₁₀ alkylene group, a C₂₋₁₀ alkenylene group and a C₂₋₁₀ alkynylene group, wherein said groups are optionally substituted with one or more substituents selected from a halogen atom, a hydroxy group, a linear or branched C₁₋₄ alkyl group, a linear or branched C₁₋₄ alkoxy group, a C₅₋₆ aryl group and a C₃₋₇ cycloalkyl group,
• L₁ and L₂ independently are selected from a direct bond, -O-, -NR^{c}-, -S-, -S(O)-, -SO₂-, -NR^{c}(CO)-, -(CO)NR^{c}-, -NR^{c}(CO)(CH₂)_{q}O-, -O(CH₂)_{q}(CO)NR^{c}-, - O(CH₂)_{q}NR^{c}-, -NR^{c}(CH₂)_{q}O-, -NR^{c}(CO)NR^{d}-, -C(O)-, -C(O)O-, -OC(O)-, -S(O)₂NR^{c}-, -NR^{c}S(O)₂-, -NR^{c}S(O)₂NR^{d}-, -C(O)NR^{c}S(O)₂- and -S(O)₂NR^{c}C(O)-, wherein R^{c} and R^{d} are independently selected from a hydrogen atom and a linear or branched C₁₋₄ alkyl group and q has a value of 0, 1 or 2,
• G and G₁ independently are selected from the group consisting of a direct bond, a C₃₋₁₀ mono- or bicyclic cycloalkyl group, a C₅-C₁₄ mono- or bicyclic aryl group, a 3-to 14-membered saturated or unsaturated mono- or bicyclic heterocyclyl group having one or more heteroatoms selected from N, S and O, a 5- to 14-membered mono- or bicyclic heteroaryl group having one or more heteroatoms selected from N, S and O and a bicyclic ring system comprising two monocyclic ring systems which are linked between each other by a covalent bond wherein said monocyclic ring systems are independently selected from a C₃₋₈ cycloalkyl group, a C₅₋₆ aryl group, a 3 to 8-membered saturated or unsaturated heterocyclyl group having one or more heteroatoms selected from N, S and O and a 5- to 6-membered heteroaryl group having one or more heteroatoms selected from N, S and O, wherein the cyclic groups independently are optionally substituted with one or more substituents selected from a halogen atom, a C₁₋₄ alkyl group, a C₁₋₄ alkoxy group, a carboxy, group, a cyano group, a nitro group, a hydroxy group, an oxo group, a trifluoromethyl group and a trifluoromethoxy group,

3. A compound according to claim 2, wherein all of k1, k2, s1, s2, 12, t1 and t2 have a value of 0.

4. A compound according to claim 3, wherein linker L has the following formula (Lb1): 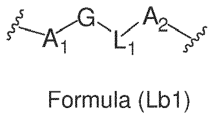 wherein A₁, A₂, L₁ and G are as defined in claim 2.

5. A compound according to claim 4, having the following formula (I): 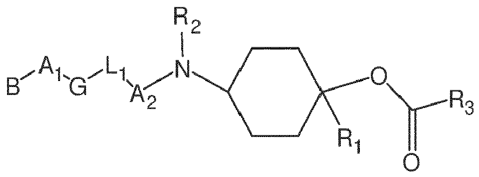 Wherein
• A1, A2, L₁ and B are as defined in claim 2,
• R₁ and R₂ independently are selected from the group consisting of a hydrogen atom and a linear or branched C₁₋₄ alkyl group,
• R₃ represents a group of formula: 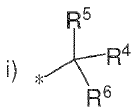 or 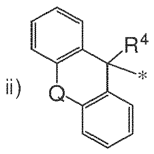 wherein:
○ R⁴ represents a hydrogen atom, a hydroxy group, a hydroxymethyl group or a linear or branched C₁₋₄ alkyl group,
○ R⁵ represents a saturated or unsaturated C₃₋₈ cycloalkyl group, C₅₋₆ aryl group, a 5- to 6- membered heteroaryl group containing at least one heteroatom selected from N, S, and O; a (C₁₋₄ alkyl)-(C₅₋₆ aryl) group, a (C₁₋₄ alkyl)-(C₃₋₈ cycloalkyl) group or a (C₁₋₄ alkyl)-(5 to 6 membered heteroaryl group containing at least one heteroatom selected from N, S, and O) group, which groups independently are optionally substituted by one or more substituents R^{a},
○ R⁶ represents a C₅₋₆ aryl group, a 5- to 6- membered heteroaryl group containing at least one heteroatom selected from N, S, and O, a saturated or unsaturated C₃₋₈ cycloalkyl group, a C₁₋₈ alkyl group, a C₂₋₈ alkenyl group, a C₂₋₈ alkynyl group, a (C₁₋₄ alkyl)-(C₅₋₆ aryl) group, a (C₁₋₄ alkyl)-(C₃₋₈ cycloalkyl) group or a (C₁₋₄ alkyl)-(5 to 6 membered heteroaryl group containing at least one heteroatom selected from N, S, and O) group, which groups independently are optionally substituted by one or more substituents R^{b},
○ R^{a} and R^{b} independently represent a halogen atom, a hydroxy group, C₁₋₄ alkyl group, C₁₋₄ alkoxy group, -SH, a C₁₋₄ alkylthio, a nitro group, a cyano group, -CO₂R', -NR'R", -C(O)NR'R", -N(R'")C(O)-R', -N(R"')-C(O)NR'R", whererin R', R" and R"' each independently represents a hydrogen atom or a C₁₋₄ alkyl group, or R' and R" together with the nitrogen atom to which they are attached from a 3 to 6 membered heterocyclic ring.
○ Q represents a direct bond, -CH₂-, -CH₂-CH₂-, -O-, -O-CH₂-, -S-, -S-CH₂-, or -CH=CH-,
○ * represents the point of attachment of R₃ to the remainder of the molecule of formula (I),
• G is selected from the group consisting of a C₃₋₁₀ mono- or bicyclic cycloalkyl group, a C₅-C₁₄ mono- or bicyclic aryl group, a 3 to 14-membered saturated or unsaturated mono- or bicyclic heterocyclyl group having one or more heteroatoms selected from N, S and O, a 5- to 14-membered mono- or bicyclic heteroaryl group having one or more heteroatoms selected from N, S and O and a bicyclic ring system consisting of two monocyclic ring systems which are linked between each other by a covalent bond wherein said monocyclic ring systems are independently selected from a C₃₋₈ cycloalkyl group, a C₅-C₆ aryl group, a 3 to 8-membered saturated or unsaturated heterocyclyl group having one or more heteroatoms selected from N, S and O and a 5- to 6-membered heteroaryl group having one or more heteroatoms selected from N, S and O, wherein the cyclic groups independently are optionally substituted with one or more substituents selected from a halogen atom, a C₁₋₄ alkyl group, a C₁₋₄ alkoxy group, a carboxy, group, a cyano group, a nitro group, a hydroxy group, an oxo group, a trifluoromethyl group and a trifluoromethoxy group,
with the proviso that when G is a phenyl group, L₁ is not one of the group selected from a direct bond, -O-, -NHC(O)-, -C(O)NH- and -NH(CO)O- group.

6. A compound according to claim 5, wherein G is selected from the group consisting of a C₅-C₆ aryl group, a 8 to 10-membered saturated or unsaturated bicyclic heterocyclyl group having one or more heteroatoms selected from N, S and O, a 8- to 10-membered bicyclic heteroaryl group having one or more heteroatoms selected from N, S and O and a C₅-C₆ aryl group linked to a ring system selected from a C₅-₆ aryl group, a C₃₋₇ cycloalkyl group and a 5- to 6-membered heteroaryl group having two or three heteroatoms selected from N, S and O, wherein the cyclic groups independently are optionally substituted with one or more substituents selected from a halogen atom, a C₁₋₄ alkyl group, a C₁₋₄ alkoxy group, a cyano group, a nitro group, a hydroxy group and oxo group, preferably, G₁ is selected from a phenyl group, 9- to 10-membered unsaturated bicyclic heterocyclyl group having one or more heteroatoms selected from N, S and O, a 9 to 10-membered bicyclic heteroaryl group having one or more heteroatoms selected from N, S and O and a C₅-C₆ aryl group linked to a ring system selected from a C₅₋₆ aryl group and a 5- to 6-membered heteroaryl group having two or three heteroatoms selected from N, S and O, wherein the cyclic groups independently are optionally substituted with one or two substituents selected from a halogen atom, a methyl group, a methoxy group, a cyano group, a hydroxy group and an oxo group.

7. A compound according to any one of claims 4 to 6, wherein L₁ is selected from the group consisting of direct bond, -NR^{c}-, -S-, -SO₂-, -C(O)-, -C(O)O-, -S(O)₂NR^{c}-, -NR^{c}S(O)₂-, -NR^{c}(CO)(CH₂)O-, -O(CH₂)(CO)NR^{c}-, -NR^{c}(CO)NR^{d}- and -CONR^{c}S(O)₂-, wherein R^{c} and R^{d} independently are selected from a hydrogen atom and a methyl group, preferably L₁ is selected from a direct bond, -NH-, -S-, -SO₂-, -C(O)-, - NR^{c}(CO)NR^{d}- and -O(CH₂)(CO)NR^{c}- group.

8. A compound according to claim 7, wherein L₁ is selected from a direct bond, -NH-, - SO₂-, -NH(CO)NH- and -O(CH₂)(CO)NR^{c}-, preferably L₁ is selected from a direct bond and -O(CH₂)(CO)NR^{c}- group.

9. A compound according to any one of claims 4 to 8, wherein -G-L₁- has the following formula: 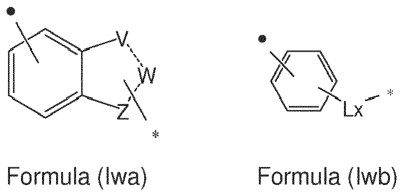 wherein
- V, W and Z are independently selected from a -N-, -C-, -S-, -O- and -C(O)-
- Lx represents a 5 to 6 membered heteroaryl group having at least one heteroatom selected from N, S and O, or Lx represents a -O-CH₂-CO-NR^{c}-, wherein R^{c} represents a hydrogen atom or a methyl group,
- * represents the point of attachment with A₂ and
- • represents the point of attachment with A₁.

10. A compound according to claim 9, wherein -G-L₁- has the following formula (Iwa): 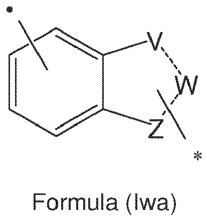 wherein V, W and Z are as defined in claim 9, preferably Z is a nitrogen atom, V represents a nitrogen atom, an oxygen atom, a carbon atom or a sulphur atom and W represents a nitrogen atom, a carbon atom or a carbonyl atom.

11. A compound according to claim 10, wherein -G-L₁ has the following formula (Iwaa): 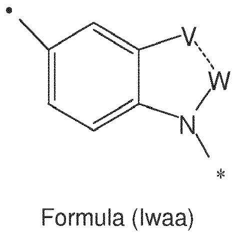 Wherein V and W are as defined in any one of claims 9 or 10.

12. A compound according to any one of claims 2 to 11, having the following formula (IA): 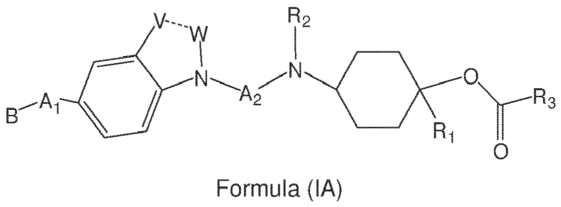 wherein R₁, R₂, R₃, A₁, A₂, V, W and B are as defined in any one of claims 5 to 11.

13. A compound according to any one of claims 2 to 12, wherein A₁ and A₂ independently represent a C₁₋₆ alkylene group optionally substituted with one or more substituents selected from a C₁₋₂ alkyl group, a C₁₋₂ alkoxy group and a phenyl group, preferably substituted with one or two substituents selected from a methyl group and a methoxy group, more preferably a methyl group.

14. A compound according to any one of claims 1 to 13, wherein B represents a group of formula (IB): 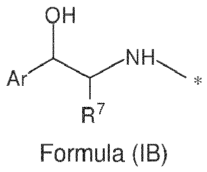 wherein:
• R⁷ is selected from the group consisting of a hydrogen atom, a linear or branched C₁₋₄ alkyl group and a linear or branched C₁₋₄ alkoxy group,
• Ar is selected from the group consisting of a C₃₋₁₀ saturated or unsaturated, mono- or bicyclic cycloalkyl group, a C₅-C₁₄ mono- or bicyclic aryl group, a 3 to 14-membered saturated or unsaturated mono- or bicyclic heterocyclyl group having one or more heteroatoms selected from N, S and O, a 5- to 14-membered mono- or bicyclic heteroaryl group having one or more heteroatoms selected from N, S and O and wherein the cyclic groups independently are optionally substituted with one or more substituents selected from a halogen atom, a cyano group, a nitro group, an oxo group, a carboxy group, a C₁₋₄ alkyl group, a C₁₋₄ alkoxy group, -CF₃, -OCF₃, -NR^{e}R^{f}, -(CH₂)ₚ-OH, -NR^{e}(CO)R^{f}, - NR^{e}-SO₂-R^{g}, -SO₂NR^{e}R^{f}, -OC(O)R^{h}, and -NR^{e}(CH₂)₍₀₋₂₎-Rⁱ, wherein p has a value of 0, 1 or 2 and wherein:
○ R^{e} and R^{f} independently represent a hydrogen atom or a linear or branched C₁₋₄ alkyl group,
○ R^{g} is selected from the group consisting of a linear or branched C₁₋₄ alkyl group, a C₆₋₅ aryl group, a saturated or unsaturated C₃₋₈ cycloalkyl, wherein the cyclic groups independently are optionally substituted with one or more substituents selected from a halogen atom, a C₁₋₄ alkyl group and a C₁₋₄ alkoxy group,
○ R^{h} is selected from a hydrogen atom,-NR^{e}R^{f} and a C₅₋₆ aryl group which is optionally substituted with one or more substituents selected from a C₁₋₄ alkyl group and a C₁₋₄ alkoxy group,
○ Rⁱ is selected from the group consisting of a C₅₋₆ aryl group, C₃₋₈ cycloalkyl group and a 3 to 8 membered saturated or unsaturated heterocyclyl group, which groups independently are optionally substituted with one or more substituents selected from halogen atom, C₁₋₄ alkyl group and a C₁₋₄ alkoxy group.

15. A compound according to claim 14, wherein Ar represents a group of formula 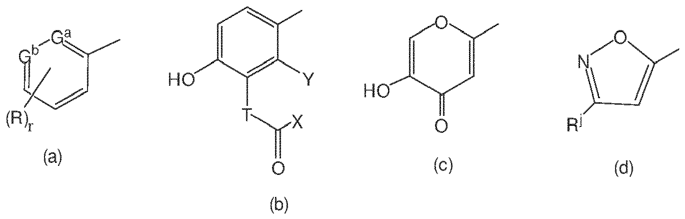 wherein
• G^{a} and G^{b} independently are selected from a nitrogen atom and a carbon atom,
• r has a value of 0, 1, 2 or 3 and
• R is seleted from the group consisting of a halogen atom, an amino group, a cyano group, a nitro group, an oxo group, a carboxy group, a C₁₋₄ alkyl group, a C₁₋₄ alkoxy group, -CF₃, -OCF₃, -(CH₂)ₚ-OH, -NH(CO)H, -NH-SO₂-R^{g}, -SO₂NH₂, -OC(O)H, -O(CO)-(4-methyl)phenyl, -O(CO)-N(CH₃)₂, -OC(O)NH₂ and - NH(CH₂)₍₁₋₂₎-Rⁱ, group, wherein p is as defined in claim 10 and R^{g} and Rⁱ independently are selected from a phenyl group optionally substituted with a substituent selected from a methyl group or a methoxy group.
• R^{j} represents a halogen atom,
• T is selected from the group consisting of -CH₂- and -NH-,
• Both X and Y represent a hydrogen atom or X together with Y form the group - CH₂-CH₂-, -CH=CH-, -CH₂-O- or -S-, wherein in the case of -CH₂-O- the methylene group is bound to the carbonyl group holding X and the oxygen atom is bound to the carbon atom in the phenyl ring holding Y,

16. A compound according to claim 15, wherein Ar represents a compound of formula (a) or (b) 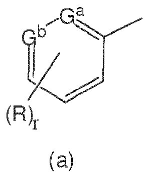 or 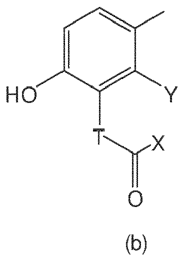 wherein:
• Both G^{a} and G^{b} represent a carbon atom,
• R is seleted from the group consisting of a halogen atom, an amino group, a cyano group, a nitro group, -(CH₂)ₚ-OH, -NH(CO)H, -NH-SO₂-CH₃, -SO₂NH₂, - OC(O)H, -O(CO)-(4-methyl)phenyl, -O(CO)-N(CH₃)₂, -OC(O)NH₂ and -CF₃ group, wherein p has a value of 0, 1 or 2,
• T represents -NH- group,
• Both X and Y represent a hydrogen atom or X together with Y form the group - CH=CH-, -CH₂-CH₂-, -CH₂-O- or -S-, wherein in the case of -CH₂-O- the methylene group is bound to the carbon atom in the amido substituent holding X and the oxygen atom is bound to the carbon atom in the phenyl ring holding Y

17. A compound according to claim 16, wherein Ar is selected from the group consisting of 3-bromoisoxazol-5-yl, 3,4-dihydroxyphenyl, 4-hydroxy-3-(methylsulfonamido)phenyl, 3,4-bis(4-methylbenzoyloxy)phenyl, 3,5-bis(dimethylcarbamoyloxy)phenyl, (5-hydroxy-6-hydroxymethyl)pyrid-2-yl, (4-amino-3,5-dichloro)phenyl, 4-hydroxyphenyl, 4-hydroxy-3-(2-hydroxyethyl)phenyl, 4-hydroxy-3-(hydroxymethyl)phenyl, [4-amino-3-chloro-5-(trifluoromethyl)]phenyl, (3-formamido-4-hydroxy)phenyl, 8-hydroxy-2-oxo-1,2-dihydroquinolin-5-yl, 8-hydroxy-2-oxo-1,2,3,4-tetrahydroquinolin-5-yl, 5-hydroxy-3-oxo-3,4-dihydro-2H-benzo[b][1,4]oxazin-8-yl and 4-hydroxy-2-oxo-2,3-dihydrobenzo[d]thiazol-7-yl, preferably 4-hydroxy-3-(hydroxymethyl)phenyl, (3-formamido-4-hydroxy)phenyl, 8-hydroxy-2-oxo-1,2-dihydroquinolin-5-yl, 8-hydroxy-2-oxo-1,2,3,4-tetrahydroquinolin-5-yl and 5-hydroxy-3-oxo-3,4-dihydro-2*H*-benzo[*b*][1,4]oxazin-8-yl.

18. A compound according to claims 16, wherein Ar represents a compound of formula (b) wherein T, X and Y are as defined in claim 16.

19. A compound according to any one of claims 1 to 18, having the following formula (IC): 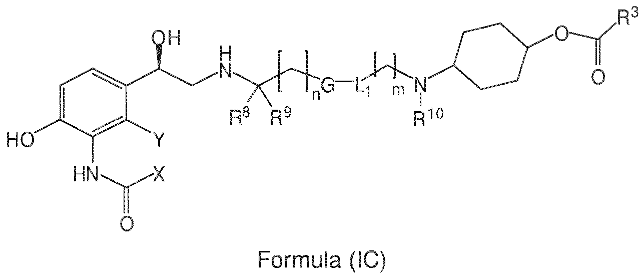 Wherein:
• R³ represents a group of formula: 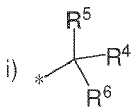 or 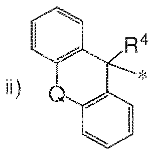 wherein:
○ R⁴ represents a hydrogen atom, a hydroxy group, a hydroxymethyl group or a linear or branched C₁₋₄ alkyl group,
○ R⁵ and R⁶ independently represent C₅₋₆ aryl group, a 5- to 6- membered heteroaryl group containing at least one heteroatom selected from N, S, and O; (C₁₋₄ alkyl)-(C₅₋₆ aryl) group, a C₃₋₈ cycloalkyl group,
○ Q represents a direct bond or a -CH₂-, -CH₂-CH₂-, -O-, -O-CH₂-, -S-, -S-CH₂-, or -CH=CH-,
○ * represents the point of attachment of R₃ to the remainder of the molecule of formula (I), • Both X and Y represent a hydrogen atom or X together with Y form the group - CH=CH-, -CH₂-CH₂-, -CH₂-O- or -S-, wherein in the case of -CH₂-O- the methylene group is bound to the carbon atom in the amido substituent holding X and the oxygen atom is bound to the carbon atom in the phenyl ring holding Y
• n has a value of 0, 1 or 2,
• m has a value of 2, 3 or 4,
• R⁸, R⁹ and R¹⁰ independently represent a hydrogen atom or a linear or branched C₁₋₄ alkyl group,
• -G-L₁- represents a group of formula: 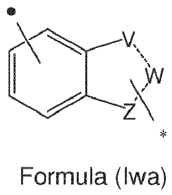 or 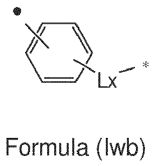
wherein
- V, W and Z are independently selected from a -N-, -C-, -S-, -O- and -C(O)-
- Lx represents a 5- to 6- membered heteroaryl group having at least one heteroatom selected from N, S and O, or Lx represents a -O-CH₂-CO-NR^{d}-, wherein R^{d} represents a hydrogen atom or a methyl group.
- * represents the point of attachment with the moiety containing the cyclohexyl group and
- • represents the point of attachment with the moiety containing the aminoethylphenol moiety,

20. A compound according to claim 19, wherein in Formula (Iwb), Lx represents a 5 to 6 membered heteroaryl group having at least one heteroatom selected from N, S and O, preferably Lx represents a pyridyl, an oxadiazolyl, an imidazolyl or a thiazolyl group, more preferably an oxadiazolyl group.

21. A compound according to claim 19 having the following formula (ID): 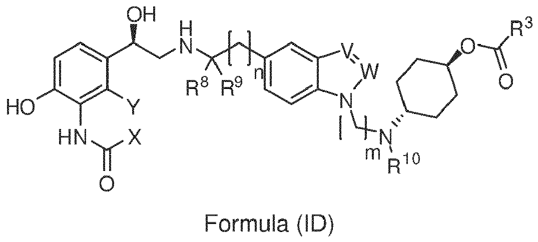 Wherein:
• V, W, X, Y, R⁸, R⁹, R¹⁰, n and m are as defined in claim 19,
• R³ represents a group of formula: 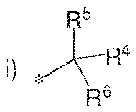 or 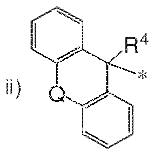 wherein:
○ R⁴ represents a hydrogen atom, a hydroxy group, a hydroxymethyl group or a linear or branched C₁₋₄ alkyl group,
○ R⁵ and R⁶ independently represents a thienyl group, a phenyl group, a benzyl group or a C₄₋₆ cycloalkyl group,
○ Q represents a direct bond or an oxygen atom,
○ * represents the point of attachment of R₃ to the remainder of the molecule of formula (I),

22. A compound qccordng to claim 19 to 21, wherein X together with Y form the group -CH=CH- or -CH₂-O-, preferably, X together with Y form the group -CH=CH-.

23. A compound according to any one of claims 19 to 22, wherein W represents a nitrogen atom or a carbonyl group, preferably W represents a nitrogen atom.

24. A compound according to any one of claims 19 to 23 wherein V represents a nitrogen atom, an oxygen atom or a sulphur atom, preferably V is a nitrogen atom or an oxygen atom.

25. A compound according to any one of claims 19 to 24 wherein V represents a nitrogen atom or an oxygen atom while W represents a carbonyl group.

26. A compound according to any one of claims 19 to 24 wherein both V and W represent a nitrogen atom.

27. A compound according to any one of claims 19 to 26 wherein n has a value 0 and/or m has a value of 3.

28. A compound according to any one of claims 19 to 27, wherein R¹⁰ represents a hydrogen atom or a methyl group and/or R⁸ and R⁹ independently represent a hygrogen atom or a methyl group, preferably R¹⁰ represents a methyl group and/or both R⁸ and R⁹ represent a hydrogen atom.

29. A compound according to any one of claims 19 to 27, wherein R₃ represents a group of formula i) wherein:
• R⁴ represents a hydrogen atom, a methyl group or a hydroxy group, preferably R^{a} represents a hydroxy group,
• R⁵ and R⁶ independently represent a thienyl group, a cyclopentyl group or a benzyl group, preferably both R⁵ and R⁶ are thienyl groups.

30. A compound according to claim 19 wherein -G-L₁- represents a group of formula: 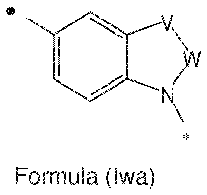 or 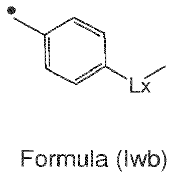 Wherein
○ V is selected from -N-, -C-, -S- and -O-,
○ W is selected from -N-, -C-, and -C(O)-,
○ Lx represents an oxadiazolyl group or -O-CH₂-CO-NR^{c}-, wherein R^{c} represents a hydrogen atom or a methyl group.
○ * represents the point of attachment with the moiety containing the cyclohexyl group and
○ • represents the point of attachment with the moiety containing the aminoethylphenol fragment,
- R⁸ and R⁹ independently are selected from a hydrogen atom and a methyl group,
- R¹⁰ represents a methyl group,
- n has a value of 0 or 1,
- m has a value of 2, 3 or 4,
- Both X and Y represents a hydrogen atom or X together with Y form -CH=CH-, -CH₂-O-, or -S- group,
- R₃ represents a group of formula: 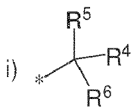 or 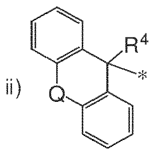 wherein:
○ R⁴ represents a methyl group or a hydroxy group,
○ R⁵ and R⁶ independently represents a thienyl group, a phenyl group, benzyl group or a cyclopentyl group,
○ Q represents a direct bond or an oxygen atom,
○ * represents the point of attachment of R³ to the remainder of the molecule of formula (I).

31. A compound according to claim 30 wherein -G-L₁- represents a group of formula (Iwaa): 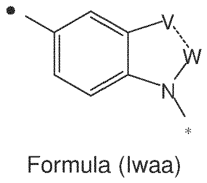 Wherein
○ W represents a nitrogen atom or a carbonyl group,
○ V represents a nitrogen or an oxygen atom,
- Both R⁸ and R⁹ represents a hydrogen atom,
- X together with Y form -CH=CH-,
- R₃ represents a group of formula i) wherein R⁴ represents a hydroxy group and both R⁵ and R⁶ represent a thienyl group.

32. A compound according to claim 1 which is one of
Trans-4-[{3-[6-({[(2R)-2-hydroxy-2-(8-hydroxy-2-oxo-1,2-dihydroquinolin-5-yl)ethyl] amino}methyl)-2-oxo-1,3-benzoxazol-3(2H)-yl]propyl}(methyl)amino]-cyclohexyl hydroxy(di-2-thienyl)acetate, dihydrofluoride,
trans-4-[{3-[5-({[(2R)-2-hydroxy-2-(8-hydroxy-2-oxo-1,2-dihydroquinolin-5-yl)ethyl]amino}methyl)-2-oxo-2,3-dihydro-1H-benzimidazol-1-yl]propyl} (methyl)amino]cyclohexyl hydroxy(di-2-thienyl)acetate dihydrofluoride,
Trans-4-[{3-[5-({[(2R)-2-hydroxy-2-(8-hydroxy-2-oxo-1,2-dihydroquinolin-5-yl)ethyl]amino}methyl)-1H-1,2,3-benzotriazol-1-yl]propyl}(methyl)amino] cyclohexylhydroxy(di-2-thienyl)acetate dihydrofluoride,
Trans-4-[{3-[5-({[(2R)-2-hydroxy-2-(8-hydroxy-2-oxo-1,2-dihydroquinolin-5-yl)ethyl]amino}methyl)-1H-indol-1-yl]propyl}(methyl)amino]cyclohexylhydroxy (di-2-thienyl)acetate dihydrofluoride,
Trans-4-[{3-[5-({[(2R)-2-hydroxy-2-(8-hydroxy-2-oxo-1,2-dihydroquinolin-5-yl)ethyl]amino}methyl)-1H-benzimidazol-1-yl]propyl}(methyl)amino]cyclohexyl hydroxy(di-2-thienyl)acetate,
Trans-4-[{3-[5-({[(2R)-2-hydroxy-2-(8-hydroxy-2-oxo-1,2-dihydroquinolin-5-yl) ethyl]amino}methyl)-1H-indazol-1-yl]propyl}(methyl)amino]cyclohexylhydroxy(di-2-thienyl)acetate,
Trans-4-[{3-[6-({[(2R)-2-hydroxy-2-(8-hydroxy-2-oxo-1,2-dihydroquinolin-5-yl)ethyl]amino}methyl)-2-oxo-1,3-benzothiazol-3(2H)-yl]propyl}(methyl)amino] cyclohexyl hydroxy(di-2-thienyl)acetate,
Trans-4-[(3-{5-[({(2R)-2-[3-(formylamino)-4-hydroxyphenyl]-2-hydroxyethyl}amino)methyl]-1H-1,2,3-benzotriazol-1-yl}propyl)(methyl)amino] cyclohexyl hydroxy(di-2-thienyl)acetate,
Trans-4-[{3-[5-({[(2R)-2-hydroxy-2-(4-hydroxy-2-oxo-2,3-dihydro-1,3-benzothiazol-7-yl)ethyl]amino}methyl)-1H-1,2,3-benzotriazol-1-yl]propyl}(methyl) amino]cyclohexyl hydroxy(di-2-thienyl)acetate,
Trans-4-[{3-[6-(2-{[(2R)-2-hydroxy-2-(8-hydroxy-2-methylene-1,2-dihydroquinolin-5-yl)ethyl]amino}ethyl)-2-oxo-1,3-benzoxazol-3(2H)-yl]propyl}(methyl)amino] cyclohexyl hydroxy(di-2-thienyl)acetate,
Trans-4-[{2-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]ethyl}(methyl)amino] cyclohexylhydroxy(di-2-thienyl)acetate,
Trans-4-[{4-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]butyl}(methyl)amino] cyclohexylhydroxy(di-2-thienyl)acetate,
Trans-4-[{3-[5-({[(2R)-2-hydroxy-2-(8-hydroxy-2-oxo-1,2-dihydroquinolin-5-yl)ethyl]amino}methyl)-1 H-1,2,3-benzotriazol-1-yl]propyl}(methyl)amino] cyclohexylcyclopentyl(hydroxy)2-thienylacetate,
Trans-4-[{3-[6-({[(2R)-2-hydroxy-2-(8-hydroxy-2-oxo-1,2-dihydroquinolin-5-yl)ethyl]amino}methyl)-2-oxo-1,3-benzoxazol-3(2H)-yl]propyl}(methyl)amino] cyclohexyl-9-methyl-9H-xanthene-9-carboxylate,
Trans-4-[{3-[6-({[(2R)-2-hydroxy-2-(5-hydroxy-3-oxo-3,4-dihydro-2H-1,4-benzoxazin-8-yl)ethyl]amino}methyl)- 2-oxo-1,3-benzothiazol-3(2H)-yl]propyl} (methyl)amino]cyclohexyl hydroxy(di-2-thienyl)acetate,
Trans-4-[(2-{5-[({(2R)-2-[3-(formylamino)-4-hydroxyphenyl]-2-hydroxyethyl} amino)methyl]-1H-indol-1-yl}ethyl)(methyl)amino]cyclohexyl 9H-fluorene-9-carboxylate,
Trans-4-[(3-{5-[({(2R)-2-[3-(formylamino)-4-hydroxyphenyl]-2-hydroxyethyl} amino)methyl]-1H-indol-1-yl}propyl)(methyl)amino]cyclohexyl 2-hydroxy-3-phenyl-2-(2-thienyl)propanoate,
Trans-4-[{3-[5-(2-{[(2R)-2-hydroxy-2-(8-hydroxy-2-oxo-1,2-dihydroquinolin-5-yl)ethyl]amino}-2-methylpropyl)-2-oxo-2,3-dihydro-1H-benzimidazol-1-yl]propyl} (methyl)amino]cyclohexyl 2,2-diphenylpropanoate,
Trans-4-[{2-[5-(2-{[(2R)-2-hydroxy-2-(8-hydroxy-2-oxo-1,2-dihydroquinolin-5-yl) ethyl]amino}-2-methylpropyl)-1H-indazol-1-yl]ethyl}(methyl)amino]cyclohexyl 2-phenyl-2-(2-thienyl)propanoate,
Trans-4-[{3-[6-(2-{[(2R)-2-hydroxy-2-(8-hydroxy-2-methylene-1,2-dihydroquinolin-5-yl)ethyl]amino}propyl)-2-oxo-1,3-benzoxazol-3(2H)-yl]propyl}(methyl)amino] cyclohexyl hydroxy(di-2-thienyl)acetate,
Trans-4-[(3-{3-[4-({[(2R)-2-hydroxy-2-(8-hydroxy-2-oxo-1,2-dihydroquinolin-5-yl)ethyl]amino}methyl)phenyl]-1,2,4-oxadiazol-5-yl}propyl)(methyl)amino]cyclohexyl hydroxy(di-2-thienyl)acetate,
Trans-4-[{2-[{[4-({[(2R)-2-hydroxy-2-(8-hydroxy-2-oxo-1,2-dihydroquinolin-5-yl)ethyl]amino}methyl)phenoxy]acetyl}(methyl)amino]ethyl}(methyl)amino]cyclohexy I hydroxy(di-2-thienyl)acetate,
Trans-4-[[2-({[4-({[(2R)-2-hydroxy-2-(8-hydroxy-2-oxo-1,2-dihydroquinolin-5-yl)ethyl]amino}methyl)phenoxy]acetyl}amino)ethyl](methyl)amino]cyclohexyl hydroxy(di-2-thienyl)acetate,
Trans-4-[(3-{3-[2-chloro-4-({[(2R)-2-hydroxy-2-(8-hydroxy-2-oxo-1,2-dihydroquinolin-5-yl)ethyl]amino}methyl)-5-methoxyphenyl]-1,2,4-oxadiazol-5-yl}propyl)(methyl)amino]cyclohexyl hydroxy(di-2-thienyl)acetate, and
Trans-4-[{2-[{[2-chloro-4-({[(2R)-2-hydroxy-2-(8-hydroxy-2-oxo-1,2-dihydroquinolin-5-yl)ethyl]amino}methyl)-5-methoxyphenoxy]acetyl}(methyl)amino]ethyl}(methyl) amino]cyclohexyl hydroxy(di-2-thienyl)acetate,
or Pharmaceutical acceptable salts or N-oxides or solvates or deuterated derivative thereof:

33. A compound according to any one of claims 1 to 32 for use in the treatment of a human or animal body by therapy.

34. A compound according to any one of claims 1 to 32 for use in the treatment of a pathological condition or disease with β2 adrenergic receptor agonist and muscarinic receptor antagonist activities, which condition or disease is preferably selected from pulmonary diseases, pre-labor, glaucoma, neurological disorders, cardiac disorders, inflammation and gastrointestinal disorders, and is more preferably asthma and or chronic obstructive pulmonary disease.

35. A pharmaceutical composition comprising a compound as defined in any one of claims 1 to 32 in association with a Pharmaceutical acceptable diluent or carrier.

36. A combination product comprising (i) a compound according to any one of claims 1 to 32; and (ii) another compound selected from a corticosteroid and a PDE4 inhibitor, for simultaneous, separate or sequential use in the treatment of the human or animal body.
